# EUROPEAN PATENT APPLICATION

(11) **EP 4 083 232 A1**
(43) Date of publication of application: **02.11.2022**
(21) Application number: 20908235.3
(22) Date of filing: 17.01.2020
(51) Int. Cl.: C12Q 1/6886, C12Q 1/6858, C12N 15/11

(54) **COMBINATION OF DNA METHYLATION BIOMARKERS, AND DETECTION METHOD THEREFOR AND KIT THEREOF**

(30) Priority: 26.12.2019 CN 201911367385
(71) Applicant: Anchordx Medical Co., Ltd., Guangzhou, Guangdong 510300 (CN)
(72) Inventor: RUAN, Weimei, Guangzhou Guangdong 510300 (CN); JIANG, Zeyu, Guangzhou Guangdong 510300 (CN); LI, Xia, Guangzhou Guangdong 510300 (CN); CHEN, Zhiwei, Guangzhou Guangdong 510300 (CN); FAN, Jianbing, Guangzhou Guangdong 510300 (CN)
(74) Representative: Grund, Martin
(86) International application number: PCT/CN2020/072770
(87) International publication number: WO 2021/128519

(57) **Abstract**

The present disclosure relates to a combination of DNA methylation markers for bladder cancer detection, which is selected from any combination of two or more sequences from SEQ ID Nos. 1 -22 or any combination of two or more completely complementary sequences to SEQ ID Nos. 1 -22. The present disclosure further relates to a detection kit for the above combination of methylation markers. In the present disclosure, the combination of the co-methylation status of multiple specific methylated regions is used to identify and analyze the occurrence of bladder cancer. The specific methylation combination is highly sensitive to identify the occurrence of bladder cancer, and the detection method is simple and feasible. In terms of designing primers, the combination of pairs of primers of the kit overcomes the shortcoming of false positive due to mismatch in the detection of a single methylation site, and takes into account of the interactions between combinations of primers and probes for the multiple methylation biomarkers.

## Description

### TECHNICAL FIELD

The present disclosure belongs to biotechnology, in particular to a combination of DNA methylation biomarkers, a detection kit and/or a detection method.

### BACKGROUND

Bladder cancer is one of the most common malignant tumors in the urinary system. New cases of bladder cancer in China was estimated to be 78,000 people/year by 2014, and the morbidity is increasing year by year. Bladder cancer is characterized with a high morbidity and high rate of recurrence. A common presenting clinical symptom of bladder cancer is hematuria, among which about 17% of patients were diagnosed with bladder cancer. At present, tests for bladder cancer diagnosis mainly include cystoscopy, urine exfoliative cytology, urinary Fish test and tumor marker detection. While cystoscopy followed by biopsy histopathology is the gold standard for the diagnosis of bladder cancer, this detection method is invasive, prone to complications, resulting in low patient compliance. The imaging examination has a limited ability for the diagnosis of a small lesion, the urine exfoliative cytology has a low sensitivity, and the urinary FISH test is complex in operation and is subjective in the interpretation of result. The existing detection of tumor markers is mainly based on the presence of specific proteins in urine, but still has a limited sensitivity and specificity due to the low content of proteins in urine.

By seeking a combination of specific DNA methylation biomarkers for bladder cancer, the detection based on multiple DNA methylation sites overcomes the problem of low detection signal from single-site DNA methylation, and improves the sensitivity and specificity of detection. Meanwhile, the detection based on DNA methylation is easy in operation and the interpretation is objective, and avoids subjective interpretation of the results and improves accuracy. Furthermore, the detection is non-invasive to avoid complications caused by cystoscopy, thus improving the patient compliance.

### SUMMARY OF THE INVENTION

An object of the present disclosure is to provide a combination of DNA methylation markers that can be used in the diagnosis of bladder cancer.

A technical solution to achieve the above object is as follow:
a combination of DNA methylation markers for bladder cancer detection is selected from any combination of two or more sequences from SEQ ID No. 1 to SEQ ID No. 22 of the co-methylated regions indicated by [CG], or is selected from any combination of two or more completely complementary sequences to SEQ ID No. 1 to SEQ ID No. 22.

Further, a combination of 2-3, 2-4, 2-5, 2-6, 2-8, 2-9, 2-10, 2-11, 2-12, 2-13, 2-14, 2-15, 2-16, 2-17, 2-19, 2-20, 2-21 or 2-22 DNA methylation markers could be selected optionally.

In some embodiments, the combination of DNA methylation markers for bladder cancer detection includes a combination of at least two sequences selected from SEQ ID No. 2, SEQ ID No. 3, SEQ ID No. 6, SEQ ID No. 13, SEQ ID No. 14, SEQ ID No. 15, SEQ ID No. 17, or a combination of at least two completely complementary sequences to SEQ ID No. 2, SEQ ID No. 3, SEQ ID No. 6, SEQ ID NO. 13, SEQ ID NO. 14, SEQ ID NO. 15, SEQ ID NO. 17.

In some embodiments, the combination of DNA methylation markers for bladder cancer detection is a combination of SEQ ID No. 1 and SEQ ID No.2 or a combination of completely complementary sequences to SEQ ID No. 1 and SEQ ID No. 2.

In some embodiments, the combination of DNA methylation markers for bladder cancer detection is a combination of SEQ ID No. 1, SEQ ID No. 2 and SEQ ID No.3, or a combination of completely complementary sequences to SEQ ID No. 1, SEQ ID No. 2 and SEQ ID No.3.

In some embodiments, the combination of DNA methylation markers for bladder cancer detection is a combination of SEQ ID Nos. 1-8 or a combination of completely complementary sequences to SEQ ID Nos. 1-SEQ ID No. 8.

In some embodiments, the combination of DNA methylation markers for bladder cancer detection is a combination of SEQ ID Nos. 1-22 or a combination of completely complementary sequences to SEQ ID Nos. 1-22.

In some of these embodiments, the combination of DNA methylation markers for bladder cancer detection comprises the following groups, or a combination of completely complementary sequences to DNA methylation markers in the following groups:

| | | | |
|---|---|---|---|
| Combination A | SEQ ID NO. 2 | | SEQ ID NO. 1 |
| Combination B | SEQ ID NO. 17 | SEQ ID NO. 20 | SEQ ID NO. 1 |
| Combination C | SEQ ID NO. 2 | SEQ ID NO. 9 | SEQ ID NO. 8 |
| Combination D | SEQ ID NO. 15 | | SEQ ID NO. 6 |
| Combination E | SEQ ID NO. 3 | SEQ ID NO. 18 | SEQ ID NO. 14 |
| Combination F | SEQ ID NO. 16 | | SEQ ID NO. 12 |
| Combination G | SEQ ID NO. 22 | SEQ ID NO. 5 | SEQ ID NO. 4 |
| Combination H | SEQ ID NO. 13 | SEQ ID NO. 10 | SEQ ID NO. 21 |
| Combination I | SEQ ID NO. 19 | SEQ ID NO. 11 | SEQ ID NO. 7 |
| Combination K | SEQ ID NO. 2 | | SEQ ID NO. 6 |
| Combination L | SEQ ID NO.3 | | SEQ ID NO. 4 |

Another object of the present disclosure is to provide use of the above combination of DNA methylation markers for the preparation of a kit for detection, diagnosis, classification, prediction, treatment monitoring, prognosis or otherwise evaluation for bladder cancer.

Another object of the present disclosure is to provide a kit for bladder cancer detection. The kit can be used for diagnosis, monitoring, concomitant diagnosis, analysis, rating, treatment and the like for a patient with bladder cancer.

A kit for identifying bladder cancer at different grades or stages comprises a reagent for detecting a co-methylation level of a combination of DNA methylation markers of at least two of SEQ ID No.2, SEQ ID No.3, SEQ ID No.5, SEQ ID No.6, SEQ ID No. 13, SEQ ID No. 14, SEQ ID No. 15, SEQ ID No. 16, SEQ ID No. 17, SEQ ID No.18, SEQ ID No.21, and SEQ ID No.22 of a co-methylated region indicated by [CG], or a co-methylation level of a combination of completely complementary sequences to SEQ ID No.2, SEQ ID No.3, SEQ ID No.5, SEQ ID No.6, SEQ ID No.13, SEQ ID No.14, SEQ ID No.15, SEQ ID No.16, SEQ ID No.17, SEQ ID No.18, SEQ ID No.21, and SEQ ID No.22.

In some embodiments, the combination of DNA methylation markers for bladder cancer detection is a combination of DNA methylation markers of SEQ ID No.6, SEQ ID No.13, SEQ ID No.16, and SEQ ID No.18 or a combination of DNA methylation markers of completely complementary sequences to SEQ ID No.6, SEQ ID No. 13, SEQ ID No.16, and SEQ ID No.18.

In some embodiments, the combination of DNA methylation markers for bladder cancer detection comprises DNA methylation markers of SEQ ID NO.6, SEQ ID NO.10, SEQ ID NO.13, SEQ ID NO. 16, and SEQ ID NO. 18 or DNA methylation markers of complementary sequences to SEQ ID NO.6, SEQ ID NO.10, SEQ ID NO.13, SEQ ID NO. 16, and SEQ ID NO. 18.

In some embodiments, the combinations of DNA methylation markers for bladder cancer detection comprises DNA methylation markers of SEQ ID NO.17 and SEQ ID NO.13 or DNA methylation markers of completely complementary sequences to SEQ ID NO.17 and SEQ ID NO.13.

In some of these embodiments, the combination of DNA methylation markers is a combination of SEQ ID NO.17, SEQ ID NO.13, and SEQ ID NO.5, or a combination of complementary sequences to SEQ ID NO.17, SEQ ID NO.13, and SEQ ID NO.5; or the combination of DNA methylation markers is a combination of SEQ ID NO.17, SEQ ID NO.13 and SEQ ID NO.22 or a combination of the complementary sequences to SEQ ID NO.17, SEQ ID NO.13 and SEQ ID NO.22.

On the other hand, provided herein is a kit for diagnosis of bladder cancer, comprising a reagent to detect methylation level of the combination of DNA methylation markers mentioned above.

In some embodiments, when fluorescence quantitative PCR is used, the kit for bladder cancer detection comprises amplification primers and fluorescent probes for each methylated region, the amplification primers and fluorescent probes being selected from:
SEQ ID NO. 23, SEQ ID NO. 45 and SEQ ID NO. 67 for SEQ ID NO.1;
SEQ ID NO. 24, SEQ ID NO. 46 and SEQ ID NO. 68 for SEQ ID NO.2;
SEQ ID NO. 25, SEQ ID NO. 47 and SEQ ID NO. 69 for SEQ ID NO.3;
SEQ ID NO. 26, SEQ ID NO. 48 and SEQ ID NO. 70 for SEQ ID NO.4;
SEQ ID NO. 27, SEQ ID NO. 49 and SEQ ID NO. 71 for SEQ ID NO.5;
SEQ ID NO. 28, SEQ ID NO. 50 and SEQ ID NO. 72 for SEQ ID NO.6;
SEQ ID NO. 29, SEQ ID NO. 51 and SEQ ID NO. 73 for SEQ ID NO.7;
SEQ ID NO. 30, SEQ ID NO. 52 and SEQ ID NO. 74 for SEQ ID NO.8;
SEQ ID NO. 31, SEQ ID NO. 53 and SEQ ID NO. 75 for SEQ ID NO.9;
SEQ ID NO. 32, SEQ ID NO. 54 and SEQ ID NO. 76 for SEQ ID NO.10;
SEQ ID NO. 33, SEQ ID NO. 55 and SEQ ID NO. 77 for SEQ ID NO.11;
SEQ ID NO. 34, SEQ ID NO. 56 and SEQ ID NO. 78 for SEQ ID NO.12;
SEQ ID NO. 35, SEQ ID NO. 57 and SEQ ID NO. 79 for SEQ ID NO.13;
SEQ ID NO. 36, SEQ ID NO. 58 and SEQ ID NO. 80 for SEQ ID NO.14;
SEQ ID NO. 37, SEQ ID NO. 59 and SEQ ID NO. 81 for SEQ ID NO.15;
SEQ ID NO. 38, SEQ ID NO. 60 and SEQ ID NO. 82 for SEQ ID NO.16;
SEQ ID NO. 39, SEQ ID NO. 61 and SEQ ID NO. 83 for SEQ ID NO.17;
SEQ ID NO. 40, SEQ ID NO. 62 and SEQ ID NO. 84 for SEQ ID NO.18;
SEQ ID NO. 41, SEQ ID NO. 63 and SEQ ID NO. 85 for SEQ ID NO.19;
SEQ ID NO. 42, SEQ ID NO. 64 and SEQ ID NO. 86 for SEQ ID NO.20;
SEQ ID NO. 43, SEQ ID NO. 65 and SEQ ID NO. 87 for SEQ ID NO.21; or
SEQ ID NO. 44, SEQ ID NO. 66 and SEQ ID NO. 88 for SEQ ID NO.22; or
primers and probes having a plurality of consecutive nucleotides which are at least 70%, 80%, 90%, 95%, or 99% identical to the above sequences.

In some embodiments, when fluorescence quantitative PCR is used, the kit for bladder cancer detection comprises amplification primers and fluorescent probes for each methylated region, the amplification primers and fluorescent probes being selected from:
SEQ ID NO. 89, SEQ ID NO. 111 and SEQ ID NO. 133 for SEQ ID NO.1;
SEQ ID NO. 90, SEQ ID NO. 112 and SEQ ID NO. 134 for SEQ ID NO.2;
SEQ ID NO. 91, SEQ ID NO. 113 and SEQ ID NO. 135 for SEQ ID NO.3;
SEQ ID NO. 92, SEQ ID NO. 114 and SEQ ID NO. 136 for SEQ ID NO.4;
SEQ ID NO. 93, SEQ ID NO. 115 and SEQ ID NO. 137for SEQ ID NO.5;
SEQ ID NO. 94, SEQ ID NO. 116 and SEQ ID NO. 138 for SEQ ID NO.6;
SEQ ID NO. 95, SEQ ID NO. 117 and SEQ ID NO. 139 for SEQ ID NO.7;
SEQ ID NO. 96, SEQ ID NO. 118 and SEQ ID NO. 140 for SEQ ID NO.8;
SEQ ID NO. 97, SEQ ID NO. 119 and SEQ ID NO. 141for SEQ ID NO.9;
SEQ ID NO. 98, SEQ ID NO. 120 and SEQ ID NO. 142 for SEQ ID NO.10;
SEQ ID NO. 99, SEQ ID NO. 121 and SEQ ID NO. 143 for SEQ ID NO.11;
SEQ ID NO. 100, SEQ ID NO. 122 and SEQ ID NO. 144 for SEQ ID NO.12;
SEQ ID NO. 101, SEQ ID NO. 123 and SEQ ID NO. 145 for SEQ ID NO.13;
SEQ ID NO. 101, SEQ ID NO. 124 and SEQ ID NO. 146 for SEQ ID NO.14;
SEQ ID NO. 103, SEQ ID NO. 125 and SEQ ID NO. 147 for SEQ ID NO.15;
SEQ ID NO. 104, SEQ ID NO. 126 and SEQ ID NO. 148 for SEQ ID NO.16;
SEQ ID NO. 105, SEQ ID NO. 127 and SEQ ID NO. 149 for SEQ ID NO.17;
SEQ ID NO. 106, SEQ ID NO. 128 and SEQ ID NO. 150 for SEQ ID NO.18;
SEQ ID NO. 107, SEQ ID NO. 129 and SEQ ID NO. 151 for SEQ ID NO.19;
SEQ ID NO. 108, SEQ ID NO. 130 and SEQ ID NO. 152 for SEQ ID NO.20;
SEQ ID NO. 109, SEQ ID NO. 13 1 and SEQ ID NO. 153 for SEQ ID NO.21;
SEQ ID NO. 110, SEQ ID NO. 132 and SEQ ID NO. 154 for SEQ ID NO.22; or
primers and probes having a plurality of consecutive nucleotides which are at least 70%, 80%, 90%, 95%, or 99% identical to the above sequences.

In some embodiments, when fluorescence quantitative PCR is used, the kit for bladder cancer detection comprises amplification primers and fluorescent probes for each methylated region, the amplification primers and fluorescent probes being selected from:
SEQ ID NO. 155, SEQ ID NO. 177 and SEQ ID NO. 199 for SEQ ID NO.1;
SEQ ID NO. 156, SEQ ID NO. 178 and SEQ ID NO. 200 for SEQ ID NO.2;
SEQ ID NO. 157, SEQ ID NO. 179 and SEQ ID NO. 201for SEQ ID NO.3;
SEQ ID NO. 158, SEQ ID NO. 180 and SEQ ID NO. 202 for SEQ ID NO.4;
SEQ ID NO. 159, SEQ ID NO. 181 and SEQ ID NO. 203 for SEQ ID NO.5;
SEQ ID NO. 160, SEQ ID NO. 182 and SEQ ID NO. 204 for SEQ ID NO.6;
SEQ ID NO. 161, SEQ ID NO. 183 and SEQ ID NO. 205 for SEQ ID NO.7;
SEQ ID NO. 162, SEQ ID NO. 184 and SEQ ID NO. 206 for SEQ ID NO.8;
SEQ ID NO. 163, SEQ ID NO. 185 and SEQ ID NO. 207 for SEQ ID NO.9;
SEQ ID NO. 164, SEQ ID NO. 186 and SEQ ID NO. 208 for SEQ ID NO.10;
SEQ ID NO. 165, SEQ ID NO. 187 and SEQ ID NO. 209 for SEQ ID NO.11;
SEQ ID NO. 166, SEQ ID NO. 188 and SEQ ID NO. 210 for SEQ ID NO.12;
SEQ ID NO. 167, SEQ ID NO. 189 and SEQ ID NO. 211 for SEQ ID NO.13;
SEQ ID NO. 168, SEQ ID NO. 190 and SEQ ID NO. 212 for SEQ ID NO.14;
SEQ ID NO. 169, SEQ ID NO. 191 and SEQ ID NO. 213 for SEQ ID NO.15;
SEQ ID NO. 170, SEQ ID NO. 192 and SEQ ID NO. 214 for SEQ ID NO.16;
SEQ ID NO. 171, SEQ ID NO. 193 and SEQ ID NO. 215 for SEQ ID NO.17;
SEQ ID NO. 172, SEQ ID NO. 194 and SEQ ID NO. 216 for SEQ ID NO.18;
SEQ ID NO. 173, SEQ ID NO. 195 and SEQ ID NO. 217 for SEQ ID NO.19;
SEQ ID NO. 174, SEQ ID NO. 196 and SEQ ID NO. 218 for SEQ ID NO.20;
SEQ ID NO. 175, SEQ ID NO. 197 and SEQ ID NO. 219 for SEQ ID NO.21; or
SEQ ID NO. 176, SEQ ID NO. 198 and SEQ ID NO. 220 for SEQ ID NO.22; or
primers and probes having a plurality of consecutive nucleotides which are at least 70%, 80%, 90%, 95%, or 99% identical to the above sequences.

In practical application, primers and probes are selected according to the combination of specific methylated regions.

In some embodiments, the kit for bladder cancer detection further comprises primers and probes for internal reference gene: SEQ ID Nos.221-223; or primers or probes having a plurality of consecutive nucleotides which are at least 70%, 80%, 90%, 95%, or 99% identical to the above sequences.

Another object of the present disclosure is to provide a method for detecting bladder cancer.

A technical solution to achieve the above object is as follow.

A method for bladder cancer detection comprises:
extracting genomic DNA and / or free DNA from a biological sample to be detected;
performing bisulfite conversion of the DNA;
detecting co-methylation of the above combination of DNA methylation markers of the bisulfite-converted DNA and a control, to obtain a methylation profile,
comparing the methylation profile of the combination of DNA methylation markers with the identifying threshold of a profile obtained from mathematical modelling based on datasets to identify the presence of bladder cancer in the biological sample.

In some embodiments, methods for co-methylation detection comprises: methylation specific PCR, DNA methylation-based chip, targeted DNA methylation sequencing, digital PCR quantitative and fluorescence quantitative PCR.

In another aspect, the present disclosure further provides a method for diagnosis, staging and classification of bladder cancer.

The method for diagnosis, staging and classification of bladder cancer comprises:,
extracting genomic DNA and / or free DNA from biological sample to be detected;
performing bisulfite conversion of the DNA;
detecting co-methylation of the above combination of DNA methylation markers for the bisulfite-converted DNA; comparing a relative number of cycles d-C_{T} of target DNA methylation marker regions and with a pre-defined threshold,
identifying grade or stage of bladder cancer of the biological sample from different sources.

In another aspect, the present disclosure further provides a method for prediction, treatment monitoring, prognosis or otherwise evaluation of bladder cancer.

The method for prediction, treatment monitoring, prognosis, or otherwise evaluation of bladder cancer comprises:
obtaining a biological sample from an individual;
extracting genomic DNA and / or free DNA from the biological sample;
performing bisulfite conversion of the DNA;
contacting the bisulfite-converted DNA with a plurality of reagents for specifically detecting co-methylation levels of the above DNA methylation markers to measure a co-methylation level of the DNA methylation markers of the biological sample;
comparing with a identifying threshold for co-methylation level obtained from mathematical modelling based on datasets to identify prediction, treatment monitoring, or prognosis of bladder cancer.

In the present disclosure, a combination of the co-methylation status of multiple specific methylated regions (markers) is used to identify the occurrence of bladder cancer, and the specific methylation combination is highly sensitive for identifying the occurrence of bladder cancer. The detection method is simple and feasible. The present inventor found that the selected combination of the multiple methylated regions has superior performance in identifying the occurrence of bladder cancer compared to co-methylation state of a single methylated region.

In the kit for detecting multiple methylated regions according to the present disclosure, the design and combination of the pairs of primers and probes play a key role for simultaneous detection of co- methylation levels of multiple methylated regions at the same time. In terms of primer sequence designs, the combination of pairs of primers of the kit overcomes mismatch problems resulting from single site detection and thus reduces false positive amplification, and takes into account of the interactions between combinations of primers and probes for the multiple methylation biomarkers. The multiple fluorescence quantitative PCR system of this kit was optimized for the reaction components, with high amplification efficiency and the improved sensitivity of the detection method.

### BRIEF DESCRIPTION OF THE DRAWINGS

Fig1: Heat map of differences in co-methylation of multiple DNA methylated regions between bladder cancer and normal tissues.
Fig2: Heat map of differences in co-methylation of multiple DNA methylated regions in the urine between a patient with bladder cancer and a normal patient.
Fig3: Bladder cancer risk scores based on DNA co-methylation combinations were significantly different between populations with and without bladder cancer.
Fig4: Comparison in co-methylation levels of the methylated region of SEQ ID NO. 8 by using three sets of primers or probes.

### DETAIL DESCRIPTIONOF THE EMBODIMENTS

In order to facilitate the understanding of the invention, a more comprehensive description about the present disclosure is given below. The present disclosure can be implemented in many different forms, and is not limited to the embodiments described herein. On the contrary, the purpose of providing these embodiments is to make the understanding of the disclosure more thorough and comprehensive.

Unless otherwise specified, the following embodiments are performed in accordance with conventional conditions, such as those described in Sambrook et al., molecular cloning: Laboratory manual (New York: Cold Spring Harbor Laboratory Press, 1989), or those as recommended by the manufacturer. The various common chemical reagents used in the embodiments are commercially available products.

Unless otherwise defined, all technical and scientific terms used in the present disclosure are the same as understood by the technician belonging to the technical field of the present disclosure. The terms used in the specification of the present disclosure are for the purpose of describing specific embodiments only and it is not intended to limit the present disclosure. The term "and/or" used in the present disclosure includes any and all combinations of one or more related items listed.

### Definitions

To facilitate understanding of this technique, a number of terms and phrases are defined below.

Throughout the specification and claims, the following terms take the meanings explicitly associated herein, unless the context clearly dictates otherwise. The phrase "in one embodiment" used herein does not necessarily refer to the same embodiments, although it may be. Furthermore, the phrase "in another embodiment" used herein does not necessarily refer to a different embodiment, although it may be. Thus, as described below, various embodiments of the present disclosure may be readily combined without departing from the scope or spirit of the present disclosure.

In addition, as used in the invention, the term "or" is an inclusive "or" operator and is equivalent to the term "and/or", unless the context clearly dictates otherwise. The term "based on" is not exclusive and allowed for being based on additional factors not described, unless the context clearly dictates otherwise. In addition, the meanings of "a", "an" and " the" include plural reference. The meaning of "in... " includes "within ..." and "On... ".

The terms "complementary" and "complementarity" refer to nucleotides (e.g., a nucleotide) or a polynucleotide (e.g., a sequence of nucleotides) associated with base pairing rule. For Example, sequence 5 '-A-G-T-3' is complementary to sequence 3 '-T-C-A-5'. Complementary can be "partial," in which only some nucleic acid bases are matched according to the base pairing rule. Alternatively, there may be "completely" or "total" complementarity between nucleic acids. The complementary degree between nucleic acid chains affects the efficiency and strength of hybridization between nucleic acid chains. This is especially important in the amplification reactions and detection methods that depend upon binding between nucleic acids.

The term "polymerase chain reaction" is used to amplify a target sequence, which consists of: introducing a large excess of two oligonucleotide primers into the DNA mixture containing the desired target sequence, followed by a precise sequence of thermal cycling in the presence of a DNA polymerase. The two primers are complementary to their respective strands of the double stranded target sequence. For amplification, the mixture is denatured and then the primers annealed with its complementary sequence within the target molecule. After annealing, the primers were extended with a polymerase so as to form a new pair of complementary chains. The steps of denaturation, primer annealing, and polymerase extension can be repeated many times (that is, denaturation, annealing, and extension constitute one "cycle" and there can be numerous "cycles") to obtain a high concentration of amplified fragments of the desired target sequence. The length of the amplified fragment of the target sequence is determined by the relative position of the primers with respect to each other, so the length is a controllable parameter. Because of the repetitive aspects of the process, the method is referred to as the "polymerase chain reaction" (" PCR "). Since the desired amplified fragment of the target sequence becomes the predominant sequence (in terms of concentration) in the mixture, it is called "PCR amplified", "PCR products" or "amplicons".

The term "nucleic acid detection assay", as used herein, refers to any method of determining the nucleotide composition of the target nucleic acid. Nucleic acid detection assay includes but is not limited to DNA sequencing methods and probe hybridization methods.

The term "amplifiable nucleic acid" refers to a nucleic acid that can be amplified by any amplification method. It is expected that "amplifiable nucleic acid" will normally comprise "sample template".

The term "sample template" refers to the nucleic acid originating from a sample that is for analysis of presence of the "target" (as defined below). In contrast, "background template" is used to refer to nucleic acids other than the sample template, which may be or may not be present in the sample. Background template is most often inadvertent ,which may be the result of carryover, or due to the presence of nucleic acid contaminants sought to be purified from the sample. For Example, nucleic acids from an organism other than those to be tested may exist as a background for the test sample.

The term "primer" refers to an oligonucleotide, whether occurring naturally as in a purified restriction digestion or is produced synthetically, that is capable of acting as a point of initiation of synthesis when placed in the conditions for induction of synthesis of product extended from primers complementary to nucleic acid chain (e.g., in the presence of nucleotides and an inducing agent such as a DNA polymerase and under proper temperature and pH). The primer is preferably single-stranded for maximum efficiency in amplification, but may alternatively be double-stranded. In the case of double chains, the primer is first treated to separate its strands before being used to prepare the extension product. Preferably, the primer is oligodeoxyribonucleotides. The primer must be sufficiently long to initiate the synthesis of the extension product in the presence of the inducer. The exact lengths of the primers will depend on many factors, including temperature, source of the primer, and the use of the method.

The term "probe" refers to an oligonucleotide (e.g., a sequence of nucleotides) whether occuring naturally as in a purified restriction digest or produced synthetically, recombinantly, or by PCR amplification , which is capable of hybridizing to another sensitive target oligonucleotide. A probe can be single - stranded or double - stranded. Probes are useful in the detection, identification, and isolation of specific gene sequences (e.g., "capture probes"). It is anticipated that in some embodiments, any probe used in the present invention may be labeled with any "report molecule" to make it detectable in any detection system.

As used in this paper, "methylation" refers to cytosine methylation at position C5 or N4 of cytosines, the N6 position of adenine, or other types of nucleic acid methylation. In vitro DNA amplified is usually unmethylated because typical in vitro DNA amplification methods do not retain the methylation pattern of the amplified template. However, "unmethylated DNA" or "methylated DNA" can also refer to amplified DNA whose original template was unmethylated or methylated, respectively.

Accordingly, as used herein, "methylated nucleotides" or "methylated nucleotide bases" refers to the presence of a methyl moiety on a nucleotide base, where the methyl moiety is not present in a recognized typical nucleotide bases. For Example, cytosine does not contain a methyl mioety in its pyrimidine ring, but 5-methyl cytosine contains a methyl moiety at position 5 of its pyrimidine ring. Therefore, cytosine is not a methylated nucleotide and 5-methylcytosine is a methylated nucleotide. In another Example, the thymine contains a methyl moiety at position 5 of its pyrimidine ring; however, for the purposes herein, thymine is not considered a methylated nucleotide when present in DNA since thymine is a typical nucleotide base of DNA.

The methylation status can be optionally represented or indicated by a "methylation value" (e.g., representing methylation frequency, fraction, ration, percent, etc.). A methylation value can be generated, for Example, by quantifying the amount of intact nucleic acid present following restriction digestion with a methylation dependent restriction enzyme, by comparing amplication profiles after the bisulfite reaction, or by comparing the sequences of bisulfite-treated and untreated nucleic acids. Thus, a value such as methylation value, represents methylation status and can be used as a quantitative indicator of methylation status across multiple copies of a locus. The level of co-methylation is indicated or showed by the methylation status of more than one methylation site, and it is defined as co-methylation when more than one methylation site is methylated within a methylated region.

As used herein, the term "bisulfite reagent" refers to a reagent in some embodiments containing bisulphite, disulphite, hydrogen sulfite or their combinations, cytosine nucleotides without methylation in the DNA treated through bisulfite reagent processing will translate into uracil, methylated cytosine and other bases remain unchanged, so that the methylated and unmethylated cytidine such as those in two nucleotide sequence of CpG may be differentiated.

The term "methylation assay" refers to any assay used to determine the methylation status of one or more CpG dinucleotide sequences within a nucleic acid sequence.

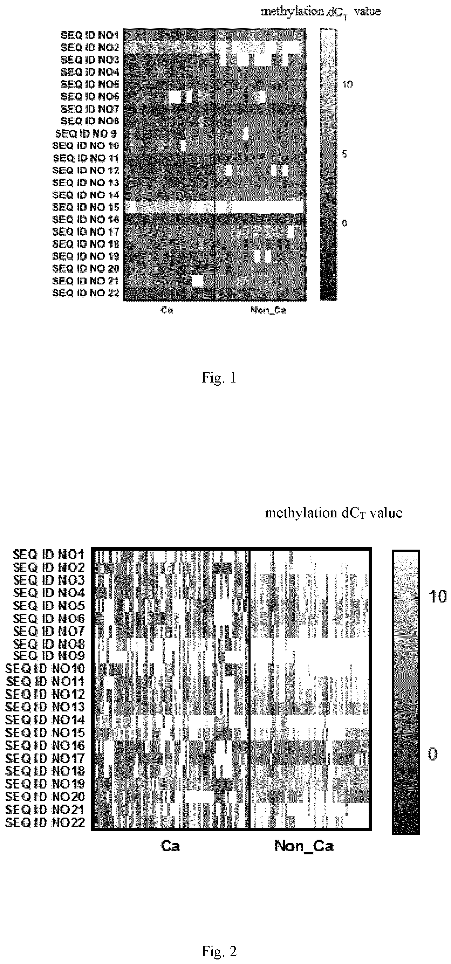

In an aspect, the present disclosure provides a combination which can be used to identify the methylation regions in a genome where bladder cancer occurs in an individual, these regions have a higher co- methylation level in DNA of tissue with bladder cancer, but a lower methylation level in a normal tissue. The level of methylation in a cancer patient is significantly different from that in a non-cancer patient (figure 1), which shows that the co-methylation levels of these methylated regions are sensitive and specific to reflect the occurrence of bladder cancer. The present disclosure further provides use of the combination of methylated regions in the diagnosis of the occurrence of bladder cancer using urine samples. Consistent with the results of tissue samples, the co-methylation levels of the above combination of methylation regions are higher in the urine DNA of a patient with bladder cancer, but lower in the urine DNA of healthy people. There is a significant difference in these two groups (figure 2), which shows that the combination of selected methylated regions in the urine DNA has highly relative signal to bladder cancer, and is superiorly sensitive to bladder cancer detection. Furthermore, using urine as a test sample, which is non-invasive, can greatly reduce the burden of patients and increase the compliance of patients to the detection.

On the other hand, the detection means include the use of methylation-specific polymerase chain reaction, nucleic acid sequencing, mass spectrometry, methylation-specific nuclease, mass-based separation or targeted capture. The detection of the above methylated regions according to the present disclosure comprises:
extracting genomic DNA and / or free DNA from a biological sample to be detected using DNA extraction kit;
Performing bisulfite conversion of the DNA;
detecting co-methylation of multiple methylated regions of the bisulfite-converted DNA.

The methods of detecting the co-methylation include methylation specific PCR (MSP), DNA methylation-based chip, targeted DNA methylation sequencing, digital PCR and quantitative fluorescence PCR.

In some embodiments, DNA (e.g. genomic DNA, such as extracted genomic DNA or processed genomic DNA) is isolated by any standard means in the field, including the use of commercially available kits.

In some embodiments, the biological sample to be detected is a biopsy material. In some cases, the biological sample is a tissue sample. In some cases, the biological sample is a biopsy sample. In some cases, the biological sample is a blood sample, including plasma, saliva, and serum. In some cases, the biological sample is a urine sample, including exfoliated cells in urine, urine sediment, and urine supernatant.

The main procedures of MSP detection method includes:
1) amplifying the co-methylated fragments in the selected target regions from the bisulfite-converted DNA respectively, by using a pair of specific primers SEQ ID NOs.23-44;
2) specific primers were used to amplifying the non-methylated fragments in the selected target regions from the bisulfite-converted DNA respectively, by using a pair of specific primers;
3) analysing the amplified products resulted from the above 1) and 2) by an agar gel electrophoresis; and
4) determining the co-methylation levels of the selected target regions according to the presence or absence of the bands or density of the bands from the electrophoresis result.

The main steps of the method for DNA methylation-based chip detection includes:
1) amplifying the whole genome from the bisulfite-converted DNA;
2) synthesizing co-methylated and non-methylated capture probes on a chip by using SEQ ID NOs. 1-22 or nucleic acid sequences which is completely complementary to SEQ ID NOs. 1-22 as target regions;
3) targeted-capturing the amplified product in 2) in the chip, and performing the labelled single-base elongation reaction; and
4) amplifying and reading sequence signals captured according to the fluorescent staining reaction, and calculating the methylation levels in the target regions.

The main steps of target DNA methylation sequencing includes:
1) amplifying the whole genome from the bisulfite-converted DNA;
2) linking the amplified product in 1) with a linker;
3) targeted-capturing the library building product in 2), wherein the capture probe used is a converted DNA sequence containing SEQ ID Nos. 1-22 or reversely and complementarily pairing sequences to SEQ ID Nos. 1-22;
4) sequencing of the capture products of 3);
5) calculating the methylation levels in the selected target regions according to the sequencing result.

The main procedures of PCR include:
1) absolute quantification of the co-methylation level in the selected target regions for the bisulfite-converted DNA by using specific primers and probes SEQ ID Nos.23-220,
2) absolute quantification of the non-methylation level in the selected target regions for the bisulfite-converted DNA by using specific primers and probes; and
3) calculating the methylation rate of each region according to the non-methylation level and the absolute quantification of the co-methylation level in each region.

The main process of fluorescence quantitative PCR is described below.

On the other hand, for a kit for detecting co-methylation level in the target methylated regions. The design and combination of the pairs of primers and probes play a key role for simultaneous detection of co- methylation levels of multiple methylated regions. In terms of primer sequence design, the combination of pairs of primers of the kit overcomes the shortcoming of false positive due to mismatch in the detection of a single methylation site, and takes into account of the interactions between combinations of pairs of primers for the multiple methylation biomarkers. The multiple fluorescence quantitative PCR system of this kit was optimized for the reaction components, up to 23 target fragments can be amplified simultaneously on the premise of ensuring the efficiency of amplifying the target fragments. The multi-fluorescence quantitative PCR reaction solution of this kit can detect the co-methylation level of up to three target regions.

The above application contains kit components: one of the pairs of primer and probe sets from 3 groups of 22 methylated target regions, which sequences are shown in tables 2-1, 2, and 3; the sets of primers and probes for the methylated regions of the internal reference gene; and multiple fluorescence quantitative PCR reaction solution of the multiple PCR reaction system.

On the other hand, the disclosure further provides a detection method for identifying the co-methylation level of the target methylated regions according to the above detection kit. This detection method can detect the co-methylation levels of up to 22 methylated regions in parallel and simultaneously process multiple samples, which has the advantages of high throughput and simple in operation.

The main procedures of the method include:
1) performing a direct fluorescence quantitative PCR reaction with the bisulfite-converted genomic DNA by using the said primers, probes and reaction reagents;
2) determining the co-methylation levels of the target regions according to a C_{T} value after corrected with an internal reference, and calculating the risk score of bladder cancer based on logistic regression.

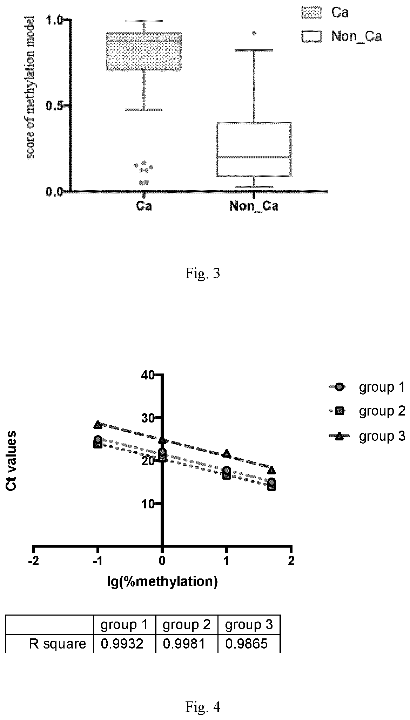

On the other hand, provided herein is a method to identify the occurrence of bladder cancer (including detection, diagnosis, classification or prediction, treatment monitoring, prognosis or otherwise evaluation of bladder cancer) based on the co-methylation level of the methylated regions. The logistic regression equation is fitted according to the co-methylation level of multiple methylated regions of the bladder cancer group and the normal group, then the risk score of bladder cancer is calculated according to the logistic regression equation. There is a significant difference between the score of bladder cancer group and that of the normal group (figure 3), resulting that the bladder cancer group can be differentiated from the control group. A statistical mathematical formula is used throughout the method of identifying the occurrence of bladder cancer according to the present disclosure, avoiding any subjectivity involving artificial judgment of a result in traditional urine FISH detection, and thus results in a more accurate, stable and reliable interpretation.

### Example1

Co-methylation in multiple methylated regions for detection, diagnosis, classification or prediction, treatment monitoring, prognosis or otherwise evaluation of bladder cancer including co-methylation of multiple methylated sites indicated by (CG) in nucleic acid sequence in table 1, as well as co-methylation of multiple methylated sites in a nucleic acid that is completely complementary in sequence to the nucleic acid indicated by (CG) in table 1.

**Table 1 Co-methylation composition of DNA methylated regions**

| SEQ ID NO | nucleic acid sequence and Co-methylation in multiple methylation sites indicated by (CG) |
|---|---|
| 1 | TAGGAAGACT[CG]GGCAC[CG]TTCAG[CG]CATTGGCTT[CG][CG]GACCCAGC[CG]C CCAGG[CG]GAT[CG]C[CG]GAAG[CG]CAAGTAG[CG]GTGTGTG[CG]CACAG |
| 2 | ATGTT[CG]G[CG]GCC[CG]GGCAC[CG][CG]AGC[CG]GC[CG]AGCTCCAGC[CG]GAGC TA[CG]TGACTA[CG]TCCACC[CG]CACCTACAGCCTGGGCAGC |
| 3 | TTGG[CG]GTCAAAGTGGCCC[CG]ACT[CG]GGATGACAATTGA[CG]GGGATCAAGGG ATTGCCCATTCTGTGCCTGTAAGAAC[CG]ATT[CG]TGCCAGAGAAACTCATCAAGTG G |
| 4 | [CG]C[CG]GGCTCCAGGGCTCC[CG][CG]CTCCAGTGGCCCAGCCTGGG[CG]GAGAGC AGAG[CG][CG]GCCCC[CG][CG]GCCC[CG][CG]GCCT[CG]AGCCC[CG] |
| 5 | CC[CG]GAGTGGGGCAGGTGT[CG]GAGCTGGGTGGGAAGCAGA[CG][CG]GTA[CG]GT GGGCAGAGGTCCCAGCCTG[CG]GGGAG[CG]CTAT |
| 6 | C[CG]GTGGTGCACCA[CG]AGGGCTACC[CG]TTTGC[CG]C[CG]C[CG]C[CG]C[CG]CAG CTGC[CG]C[CG]C[CG]C[CG]CCAGC[CG]CTGCAGCCATG |
| 7 | [CG]GGAACTGAGTGCTGGCC[CG]GGAGACCCTC[CG]GAGAGCT[CG][CG]GGCT[CG] GCCT[CG]GCCT[CG]GCCT[CG]GCCTT[CG]GC[CG][CG]GTTAC[CG]AAACACAGA[CG] GTAGACT |
| 8 | CTCC[CG][CG]CCCC[CG]CCCC[CG][CG]TTC[CG]GCCTGGCCTG[CG]GGATT[CG]GGC[ CG]AGGCAACTGCAGGGA[CG]GGGCACCCCTCCTGCTCC |
| 9 | TGGAGAGGGGTCATC[CG]CCC[CG]GAAC[CG]A[CG]TGAG[CG][CG]GGGC[CG]GCC[ CG]TGGAGG[CG]GCTGAGGGATCCCCCACTTCCAGCC[CG]CC[CG] |
| 10 | GCAGCAGCTGCAGGAAG[CG]GACT[CG]G[CG]GAAAGGAGCCC[CG]GAGGGGAACT GAGTGCCTTCAGCCAGGCA[CG]TT[CG]GGGAGACAG[CG] |
| 11 | TGC[CG]CAAAATT[CG]CAGA[CG]AAGGGCTTGTAGCC[CG][CG]TGGATG[CG]GATA TG[CG]TGTTGAG[CG]TGGAGCTG[CG]GTTGAA[CG]CTTTGC[CG] |
| 12 | TGCTACCCCAGC[CG]TGTCC[CG]CTC[CG]GAGACCCCAGGG[CG]C[CG]GGACCCATC TGC[CG]CT[CG]C[CG]GC[CG]GAGGCTACCAGGAGCAGGAGCAGCAG[CG]C[CG]CC[ CG]CAGTAG |
| 13 | [CG]GCCTTC[CG]GTGGGGCACCAAAAGGAAGCCTCCT[CG]GCCCCTGG[CG]ACC[C G]GTGACTTGCAG[CG]G[CG]TGTGATTAATCTTCCACAGCTGT[CG]TGCCCCATCCAC TTGAG |
| 14 | GCAAC[CG]GCAG[CG]TCCAGCTCC[CG]CACCT[CG]CTGCACAT[CG]CACCTGAGCCC [CG]C[CG][CG]AC[CG]CAT[CG][CG]CT[CG]CTG[CG]ACCCATTCAGACCC |
| 15 | CTTCAGCTGCCCT[CG]ATTTGCTCCA[CG]CCTGC[CG]GCCAGAGCCTCC[CG]G[CG] TTTCTTC[CG]CCCCAG[CG]GAGTG[CG]CTGGGG[CG][CG]CCAGGGCTAGGCC[CG]C[ CG]GAGGAG[CG][CG]TC |
| 16 | [CG]TGTCTGAGGCT[CG][CG]GGCAACTGGAACTGAGAGTCTGAGTTGGCCT[CG][CG ]GGAGC[CG]CCAGAAGGGTG[CG]GGCTG[CG]TGTGGCAGAGTAGGAGCACTGT |
| 17 | T[CG]GCAGTGGCCACCACATCTGGTTCT[CG]TTAACTTTCTAAGGCAG[CG]GC[CG] CTGGAGCAG[CG]GGGCTGG[CG]GGGTAAAAGCTC |
| 18 | [CG][CG]GCCAC[CG]CC[CG]TTCATCACC[CG][CG][CG]CATCTGGGCTGGCAC[CG]GG [CG]AAGAAT[CG]TG[CG]GGTCTGGGAC |
| 19 | AGATTG[CG][CG]GAGCCCA[CG][CG]ATCCCTGGGA[CG]C[CG]GAGACAA[CG]GGGC TCTTGGGAAGG[CG][CG]GAGCC[CG]GGGAAGC[CG]GGGATGTG[CG][CG]TGAGC[C G]TGCC[CG]CAGGGTC |
| 20 | GGAG[CG]TG[CG]GGCAG[CG]CCCC[CG]AACCCTAG[CG]CAGCCCAGGAAG[CG]GT[ CG]GAGGAGACTGTCCTGGC[CG][CG]GTGGCAGCCCCATC[CG]GAGTG |
| 21 | AAAACTGATC[CG]TGTCCTGCATGTTGGCAGCAGACAACCTTCCTTGCTGCTGAGCT GTCC[CG]GGTGGCTTCAC[CG][CG]GCTGGGGAATC[CG]AGCCATTCC |
| 22 | GGAGAGAAAGTCCTATCTGCAGCAGC[CG]AATGGTCCCCATTC[CG]GTAATGGGA[C G]G[CG]GGAGCATTTGGGAGGA[CG][CG]ATTCTAAAGAGAG[CG] |

EXAMPLE2 A co-methylation test kit for detection, diagnosis, classification or prediction, treatment monitoring, prognosis or otherwise evaluation of bladder cancer, including pairs of specific primers and probes for co-methylation of multiple methylated regions, as shown in table 2:

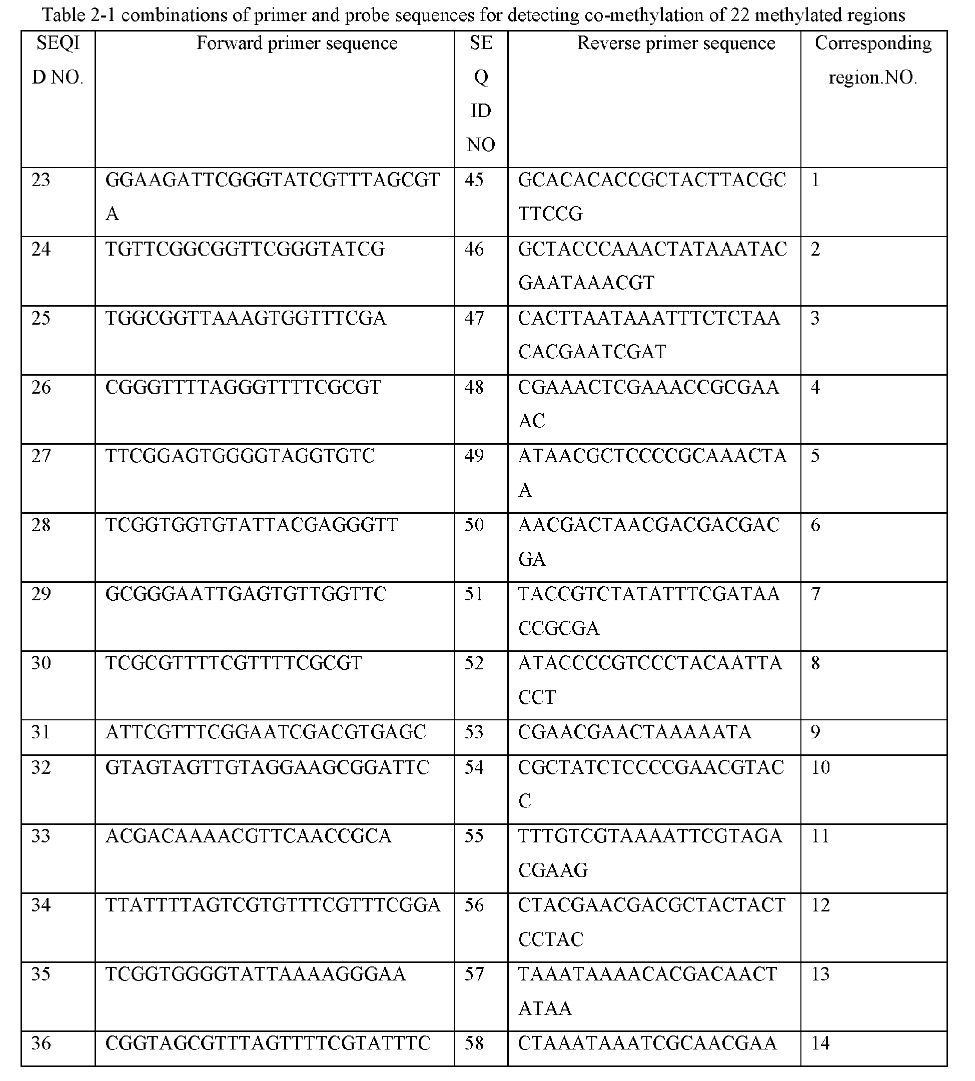

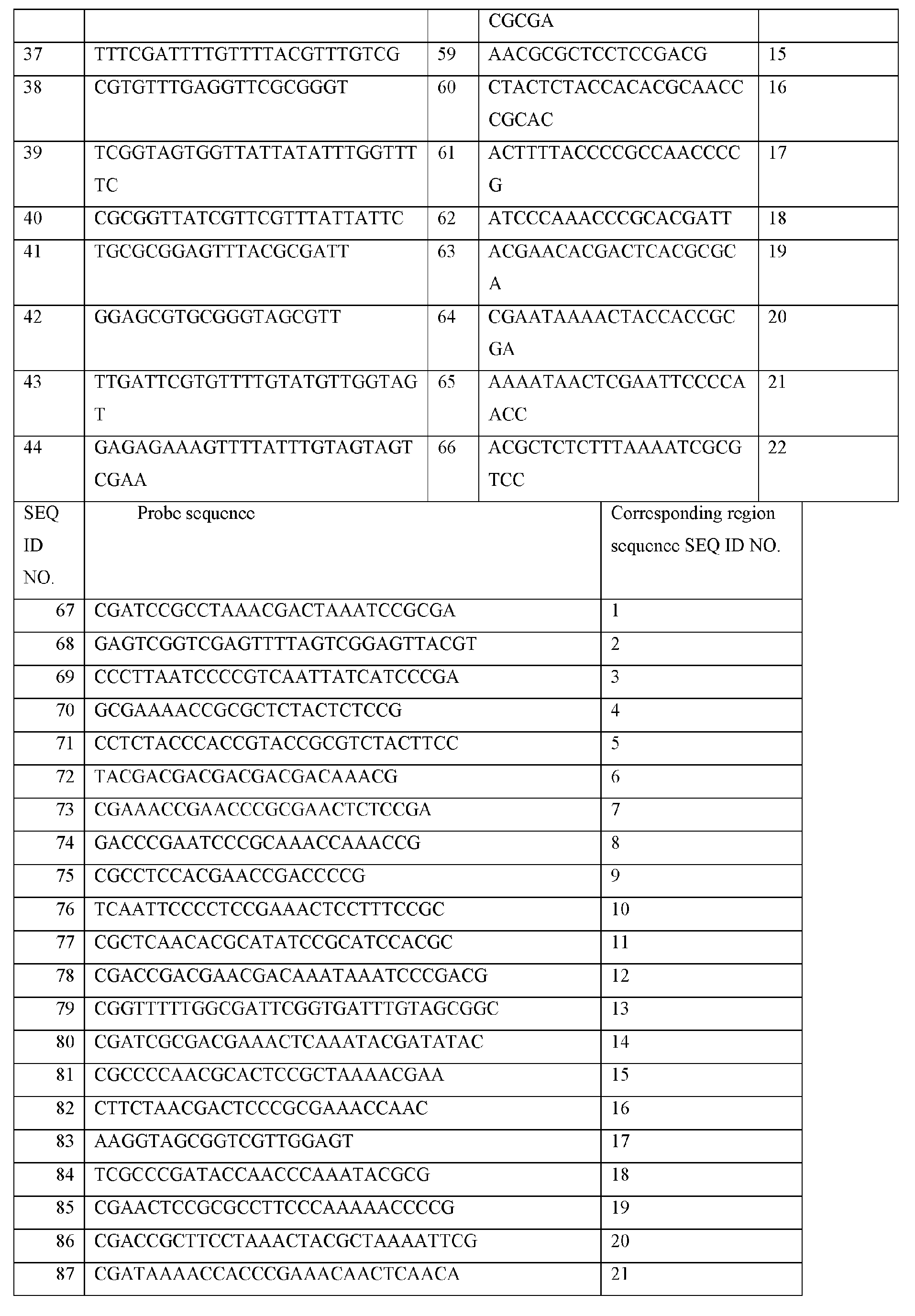

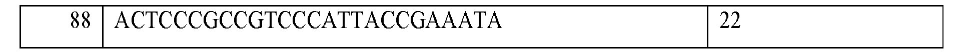

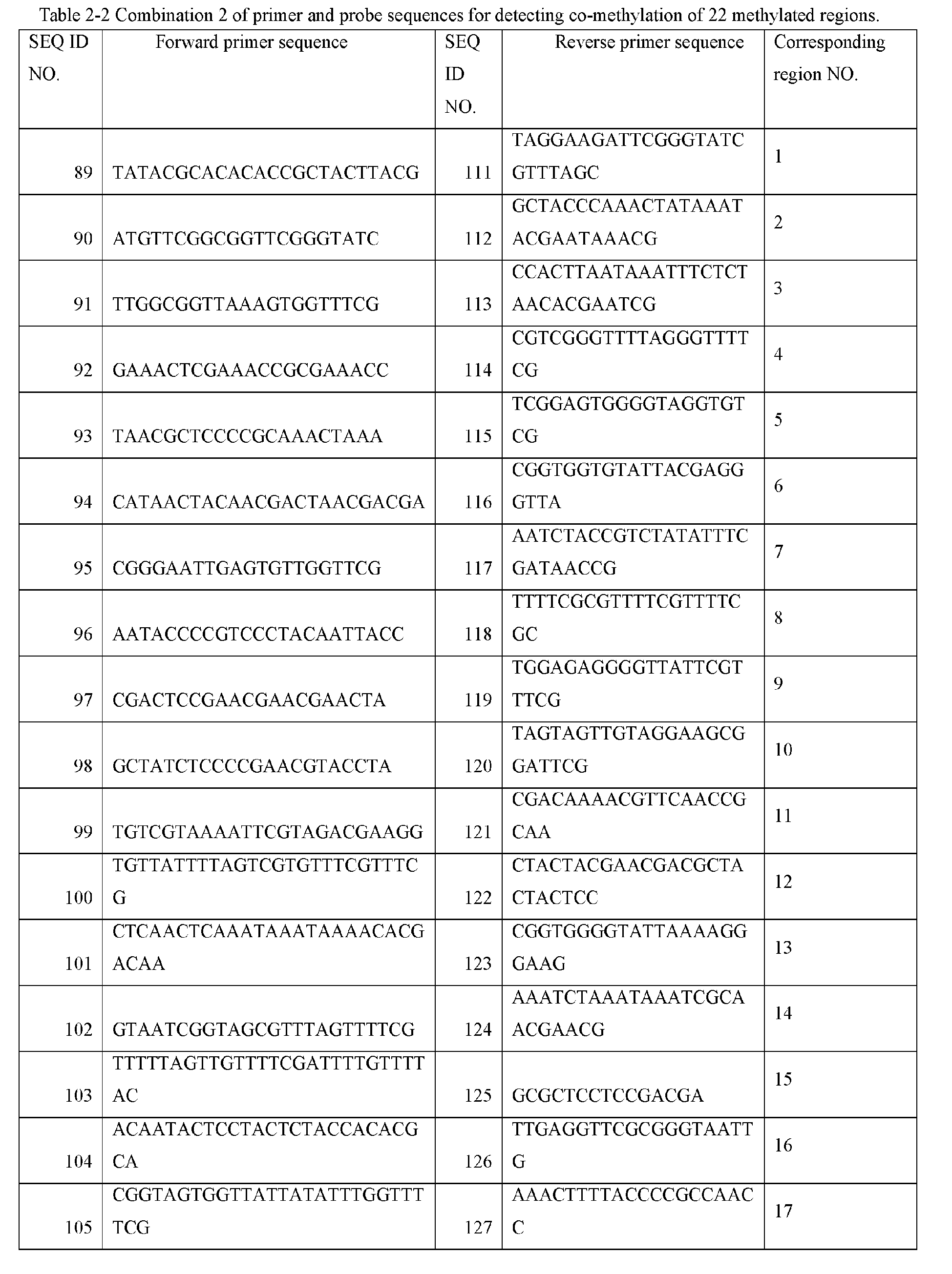

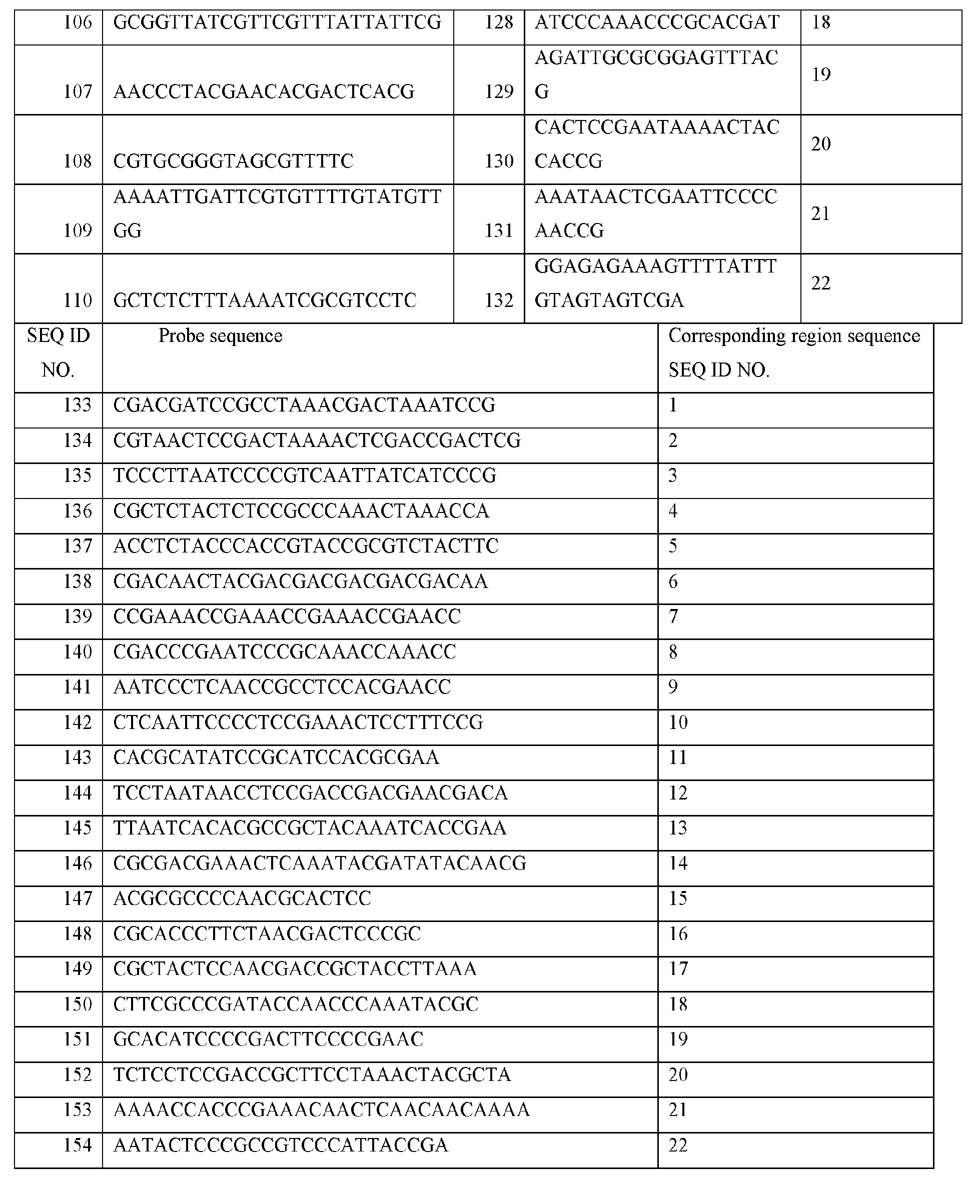

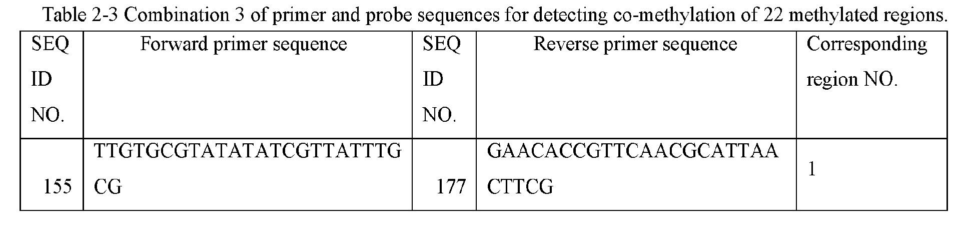

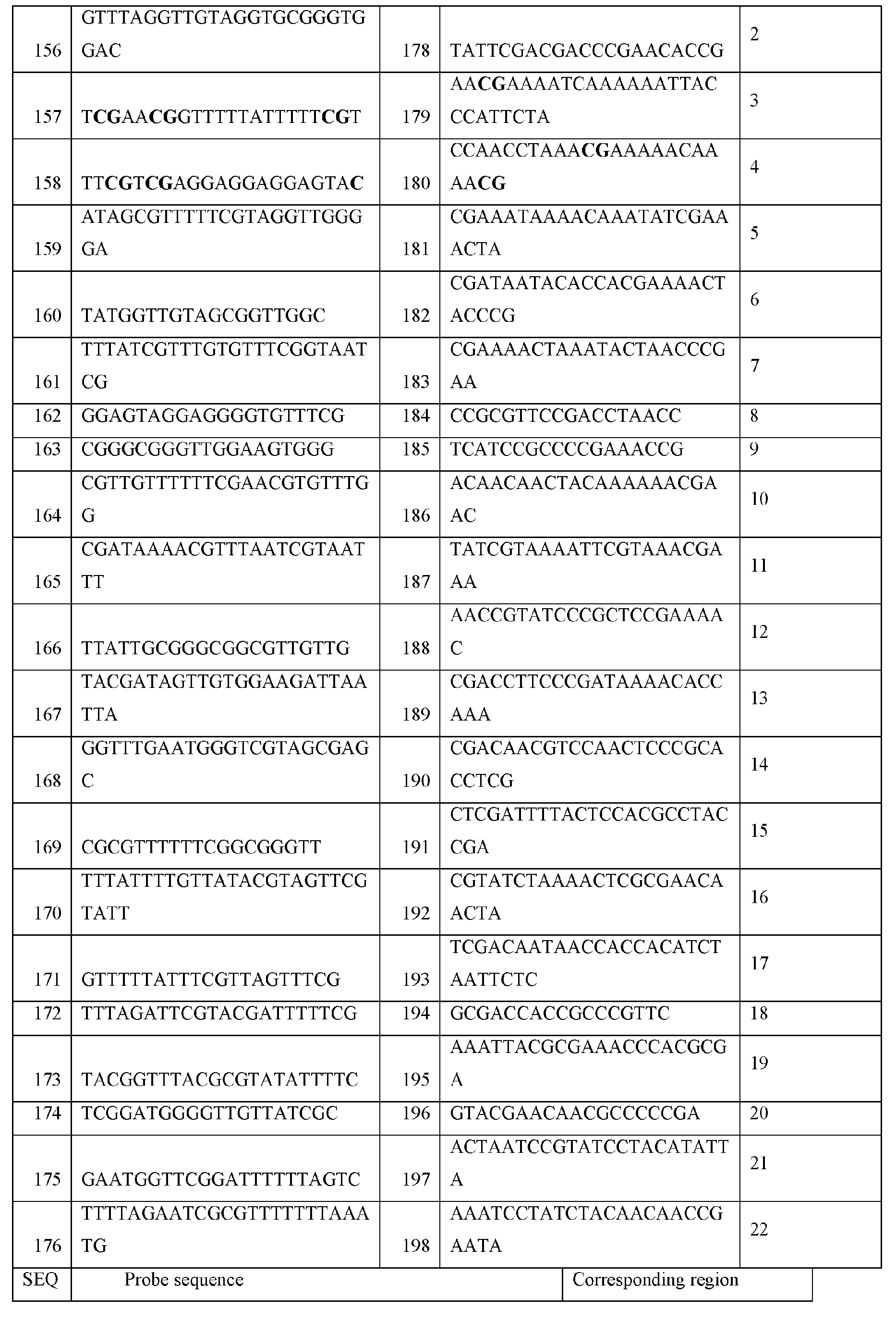

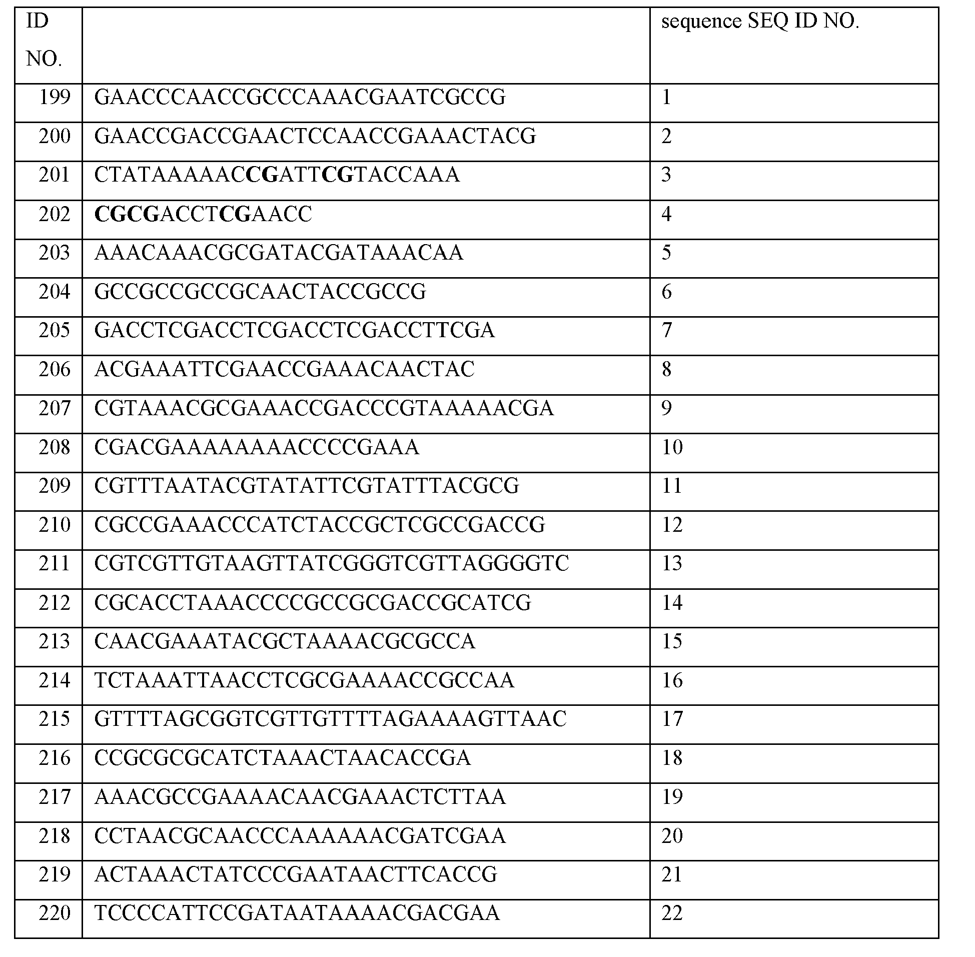

In practical application, primers and probes are selected according to the combination of specific methylated regions.
primers and probe for internal reference

| Forward primer sequence | Reverse primer sequence | probe |
|---|---|---|
| GTGATGGAGGAGGTTTAGT AAGTT(SEQ ID NO.221) | CCAATAAAACCTACTCCTCC CTTAA(SEQ ID NO.222) | ACCACCACCCAACACACAATAACA AACACA(SEQ ID NO.223) |

The kit should include one of the three combinations of the primer and probe for PCR amplification (probe fluorescent label can be FAM fluorescent label, VIC fluorescent label, NED fluorescent label and the like), the three combinations have similar performance in detection of the 22 regions. Take the detection of co-methylation level of the methylated region of SEQ ID NO. 8 as an Example, the co-methylation level of the regions of a series of standard products (Qiagen company) was detected according to the detection method of Example 3 by using a pair of primers from each of the three combinations, as shown in figure 4. The linear relationships of the primers and probes of combinations 1, 2 and 3 for the detection of the co-methylation in this regions were 0.993, 0.998 and 0.987, respectively. There was no significant difference in the linear relationships. The linear fitted equations had a slope of -3.73, -3.66 and -3.83, respectively, from which it can be determined that their amplification efficiency was similar and There was no significant difference.

The primers herein were purchased from Invitrogen company, the multiple PCR reaction reagent was purchased from Thermo Fisher company, and the multiple fluorescence quantitative PCR reagent was purchased from Qiagen company or Bio-rad company or Novazan company.

### Example 3

detection of the co-methylation of 2 or 3 DNA methylated regions by multiple fluorescence quantitative PCR. The commercial completely methylated (positive control) and non-methylated (negative control) standard products (purchased from QIAGEN) were used to detect the co-methylation of 2 or 3 DNA methylated regions for the 22 methylated regions (SEQ ID NO. 1-22).

The specific process is as follows:
1. DNA extraction
DNA extraction kit was purchased from QIAGEN company and DNA extraction was carried out according to the instruction for the extraction kit.
2. DNA conversion with bisulfite
DNA bisulfite conversion kit was purchased from Zymo and DNA bisulfite conversion was carried out according to the instructions of the kit.
3. Multiplex PCR amplification
Pairs of primer (primer sequences shown in table 2 ) for 22 methylated regions (SEQ ID NO. 1-8) were used to conduct multiplex PCR in a reaction well to amplify the target sequences containing the target regions. The product had a size of about 70-130bp.
1) The PCR primer mixture with a single primer having a concentration of 5µM (per primer) was prepared, which contained forward and reverse primers for each methylated region for the multiple reactions, all in one reaction well.
2) Preparation of PCR mixture: PCR mixture was prepared according to table 3, without addition of DNA therein.

**Table 3 scheme for preparation of PCR mixture.**

| Reagent | final concentration | volume (µL) |
|---|---|---|
| DEPC water | / | 18.5 |
| 5 x PCR Buffer | 1X | 10.0 |
| 25mM MgCl₂ | 0.25mM | 0.5 |
| 25mM dNTP mixture | 250µM | 0.5 |
| 5µM Primermixture | 0.5µM | 5.00 |
| 5U/µl Taq enzyme | 2.5 Unit | 0.5 |
| volume [µl] | / | 35.00 |

3) Adding DNA samples: into the PCR reaction well, added was 35µL PCR mixture, followed by the converted DNA which had a loading amount of 25ng before DNA conversion; the PCR reaction system had a total volume of 50µL. Eddy oscillation and centrifugation.
4) procedure for the PCR reaction: 98°C for 30 seconds; 20 cycles of 98°C for 15 seconds, 60°C for 15 seconds, 72°C for 15 seconds; and 72°C for 5 minutes. Product was stored at 4°C for use.
4. Multiplex fluorescence quantitative PCR
1) primers and probes for 22 methylated regions (see sequences listed in table 2), and primers and probes for internal reference for each methylation region were prepared as sets of mixture at a concentration of 10 µM for primer and of 5 µM for probe respectively. In 22 sets of mixture for 22 methylated regions, sets of mixture for 2 or 3 methylated regions can be mixed at an equal ratio. Some combinations for three methylated regions were listed in table 4:

**Table 4 Mixture combination scheme of primer and probe for 22 methylated regions (SEQ ID NO. 1-22) (any 2 or 3 methylated regions in the combination can be optionally selected)**

| combination scheme | FAM labeled fluorescent channel | VIC labeled fluorescent channel | NED labeled fluorescent channel |
|---|---|---|---|
| combination A | SEQ ID NO. 2 | internal reference | SEQ ID NO. 1 |
| combination B | SEQ ID NO. 17 | SEQ ID NO. 20 | SEQ ID NO. 1 |
| combination C | SEQ ID NO. 2 | SEQ ID NO. 9 | SEQ ID NO. 8 |
| combination D | SEQ ID NO. 15 | internal reference | SEQ ID NO. 6 |
| combination E | SEQ ID NO. 3 | SEQ ID NO. 18 | SEQ ID NO. 14 |
| combination F | SEQ ID NO. 16 | | SEQ ID NO. 12 |
| combination G | SEQ ID NO. 22 | SEQ ID NO. 5 | SEQ ID NO. 4 |
| combination H | SEQ ID NO. 13 | SEQ ID NO. 10 | SEQ ID NO. 21 |
| combination I | SEQ ID NO. 19 | SEQ ID NO. 11 | SEQ ID NO. 7 |
| combination J | | internal reference | SEQ ID NO. 7 |
| combination K | internal reference | internal reference | SEQ ID NO. 6 |
| combination L | SEQ ID NO.3 | internal reference | SEQ ID NO. 4 |

2) preparation of the multiplex qPCR reaction solution: according to the combination scheme in table 4, primer and probe mixture for the selected 2 or 3 methylated regions was mixed at an equal ratio to prepare PCR mixture, respectively, without addition of DNA therein.

**Table 3 scheme for preparation of PCR mixture.**

| Reagent | volume (µL) |
|---|---|
| DEPC water | 1.5-2 |
| 2 X PCR Master Mix | 5.00 |
| primers and probes for 2 or 3 marker | 0.5 (each set) |
| volume[µl] | 8.00 |

3) Adding DNA samples: into the PCR reaction well, added was 8 µL PCR mixture, followed by 2µL product of the multiplex PCR which has been subjected to two-fold dilution; the PCR reaction system had a total volume of 10 µL. Eddy oscillation and centrifugation.
4) Procedure for fluorescence quantitative PCR reaction: 95°C for 5 minutes; 95°Cfor 20 seconds, 62°C for 60 seconds, and Fluorescence signals were collected at 62°C, 40 cycles.
5. Data analysis
The co-methylation level of the 22 methylated regions was identified by using commercial completely methylated (positive control) and non-methylated DNA (negative control) standard products according to the mixed mode as shown in table 4 (combinations A - L) in multiplex fluorescence quantitative PCR, and compared with the C_{T} value from fluorescence quantitative PCR assay on a single methylation site in the 22 methylated regions (single quantity), the negative control in all combinations and single quantity was undetectable, and the C_{T} values for the positive control were shown in table 5 (notice: combination is abbreviated to "Com" and internal reference is abbreviated to "inte ref." in table 5):

| SEQ ID NO | Com A | Com B | Com C | Com D | Com E | Com F | Com G | Com H | Com I | Com J | Com K | Comb L | Single quantity |
|---|---|---|---|---|---|---|---|---|---|---|---|---|---|
| 1 | 20.29 | 21.38 | | | | | | | | | | | 18.04 |
| 2 | 23.12 | | 22.16 | | | | | | | | 23.17 | | 25.19 |
| 3 | | | | | 16.88 | | | | | | | 18.36 | 15.92 |
| 4 | | | | | | | 14.86 | | | | | 14.65 | 13.85 |
| 5 | | | | | | | 14.97 | | | | | | 13.95 |
| 6 | | | | 16.64 | | | | | | | 15.96 | | 17.76 |
| 7 | | | | | | | | | 14.50 | 14.13 | | | 15.83 |
| 8 | | | 15.25 | | | | | | | | | | 16.28 |
| 9 | | | 19.89 | | | | | | | | | | 19.51 |
| 10 | | | | | | | | 16.30 | | | | | 17.71 |
| 11 | | | | | | | | | 21.78 | | | | 20.63 |
| 12 | | | | | | 17.89 | | | | | | | 17.22 |
| 13 | | | | | | | | 16.92 | | | | | 16.78 |
| 14 | | | | | 17.98 | | | | | | | | 16.21 |
| 15 | | | | 24.63 | | | | | | | | | 25.50 |
| 16 | | | | | | 13.64 | | | | | | | 13.92 |
| 17 | | 18.05 | | | | | | | | | | | 17.54 |
| 18 | | | | | 21.08 | | | | | | | | 20.47 |
| 19 | | | | | | | | | 21.52 | | | | 20.71 |
| 20 | | 17.02 | | | | | | | | | | | 16.02 |
| 21 | | | | | | | | 17.50 | | | | | 16.98 |
| 22 | | | | | | | 18.15 | | | | | | 17.74 |
| inte ref. | 18.83 | | | 17.95 | | | | | | 18.21 | 17.96 | 20.29 | 20.60 |

As shown in Table 5, according to the combination scheme, the primes and probes for any two or three of the methylated regions in the combination were mixed to perform a multiplex fluorescence quantitative PCR, the resulted C_{T} values are similar to those quantified for a single region, with no significant differences. It was estimated that no mutual interference in the amplification efficiency occurs among the 22 methylated regions in the combination scheme of multiplex fluorescence quantity, the quantitative performance was the same as that in single region quantity, and the simultaneous quantitative detection for 2 or 3 methylated regions can be achieved. Example 4 Detection of co-methylation of 22 methylated regions in bladder cancer cell lines, bladder cancer tissues and pericarcinomatous tissues

The co-methylation of 22 methylated regions was detected for DNA from bladder cancer cell lines 5637, T24 (purchased from Shanghai Institute of cell) and UM - UC - 3 (purchased from the sigma), and 16 bladder cancer tissues, corresponding normal pericarcinomatous tissues respectively, using the detection method described in Example 3, to verify the application of these methylated regions in the diagnosis of bladder cancer. The C_{T} values for each methylated region from the detection were corrected by the C_{T} value for internal reference, and the relative cycle number of the target regions was obtained as d-C_{T} = C_{T} (target region) - C_{T} (internal reference). If the target regions are undetectable, the relative cycle number of the target regions is given as d- C_{T} = 35. The pathological composition information of 18 patients with bladder cancer is shown in table 6.

**Table 6 The pathological composition information of 18 patients with bladder cancer.**

| | cases | Proportion (%) | Average age (scope) |
|---|---|---|---|
| Grade | | | |
| Low grade | 3 | 18.8% | 60 (48-78) |
| High grade | 13 | 81.2% | 59 (40-77) |

| stages | | | |
|---|---|---|---|
| Ta | 3 | 18.8% | 67 (63-78) |
| T1 | 8 | 50% | 62 (43-73) |
| T2-T4 | 5 | 31.2% | 52 (40-77) |

| Invasiveness | | | |
|---|---|---|---|
| muscle-invasive (T2-T4) | 5 | 31.2% | 52 (40-77) |
| non-muscle-invasive (Ta-T1) | 11 | 68.8% | 63 (43-78) |

The median relative cycle number d-C_{T} values of the co-methylation level of the 22 methylated regions of the bladder cancer cell lines, bladder cancer tissues, adjacent normal tissues and positive controls were shown in table 7. The methylation heat map of all tissue samples according to the d-C_{T} values for 22 methylated regions is shown in Figure 1.

**Table 7 median relative cycle number d-C_{T} values for the co-methylation level of the 22 methylated regions of bladder cancer cell lines, bladder cancer tissues and normal pericarcinomatous tissues.**

| | Bladder cancer cell lines | | | | | |
|---|---|---|---|---|---|---|
| | 5637 | T24 | UM-UC-3 | Bladder cancer tissues | normal pericarcinomatous tissues | Positive control |
| SEQ ID NO. 1 | -2.99 | -1.48 | -2.70 | 2.04 | 5.22 | 2.19 |
| SEQ ID NO. 2 | 35 | -3.79 | 35 | 7.45 | 13.06 | 4.17 |
| SEQ ID NO. 3 | -4.22 | -3.55 | -3.59 | 0.90 | 6.28 | -1.03 |
| SEQ ID NO. 4 | -0.73 | -0.93 | -0.75 | 1.45 | 4.13 | -3.89 |
| SEQ ID NO. 5 | -2.97 | -2.11 | -1.78 | 0.29 | 2.72 | -3.68 |
| SEQ ID NO. 6 | -0.43 | -2.04 | -1.17 | 1.98 | 4.83 | -2.34 |
| SEQ ID NO. 7 | -3.17 | -3.70 | -3.39 | -1.46 | 0.14 | -4.33 |
| SEQ ID NO. 8 | -2.42 | 0.47 | -2.79 | 0.13 | 3.75 | -3.40 |
| SEQ ID NO. 9 | -1.76 | -2.56 | -0.98 | 0.34 | 3.93 | 1.24 |
| SEQ ID NO. 10 | -4.18 | -4.48 | -3.47 | 4.10 | 3.84 | -2.34 |
| SEQ ID | -3.79 | -4.70 | -3.70 | 0.32 | 2.44 | 3.14 |
| NO. 11 | | | | | | |
| SEQ ID NO. 12 | -0.33 | 0.34 | 0.90 | -0.21 | 4.37 | -0.76 |
| SEQ ID NO. 13 | 35 | 35 | 35 | 0.90 | 3.43 | -1.72 |
| SEQ ID NO. 14 | 0.95 | 0.09 | 0.98 | 2.16 | 5.01 | -0.66 |
| SEQ ID NO. 15 | 5.08 | 9.75 | 8.66 | 10.70 | 16.96 | 5.98 |
| SEQ ID NO. 16 | -4.86 | -5.13 | -3.65 | -0.50 | 0.45 | -5.01 |
| SEQ ID NO. 17 | -3.46 | -3.21 | -2.55 | 2.66 | 6.52 | -0.60 |
| SEQ ID NO. 18 | -2.39 | -3.62 | -2.63 | 2.10 | 3.51 | 2.43 |
| SEQ ID NO. 19 | -0.31 | -1.64 | -1.49 | 0.76 | 3.85 | 2.87 |
| SEQ ID NO. 20 | 35 | -2.09 | -0.33 | 1.28 | 4.18 | -1.63 |
| SEQ ID NO. 21 | -0.38 | -0.61 | 0.54 | 2.34 | 5.23 | -1.14 |
| SEQ ID NO. 22 | -3.81 | -4.96 | -3.73 | -0.48 | 1.82 | -0.50 |

As shown in figure 1 and table 7, The median cycle number d-C_{T} values for the co-methylation level of the 22 methylated regions of the bladder cancer cell lines and bladder cancer tissues approached that of positive control, and was lower than that of normal pericarcinomatous tissues, showing a statistically significant difference (p < 0.005). As a smaller d-C_{T} value describes a higher co-methylation level, it can be concluded that the co-methylation level of the selected 22 methylated regions, is significantly higher in bladder cancer cell lines and bladder tissues, positively correlated with the development of bladder cancer, and can be used as a biomarker for identifying the occurrence of bladder cancer.

In addition, according to analysis on the d-C_{T} value for 22 methylated regions at different levels and stages according to bladder cancer tissue samples at different levels and stages, it was found that different grade or different stages can be significantly differentiated in some methylated regions (p < 0.05), which demonstrates that these methylated regions and their combinations could be further used as biomarkers for identifying the bladder cancer at different grades or different stages. The methylated regions are listed in table 8, and the values as shown are the median d-C_{T} values for these methylated regions of respective groups (and interquartile range of these d-C_{T} values).

**Table 8 Median d-C_{T} values (interquartile range, IQR) for different methylated regions of bladder cancer at different grades and stages**

| | SEQ ID NO. 6 | SEQ ID NO. 10 | SEQ ID NO. 21 |
|---|---|---|---|
| Grade | | | |
| Low grade | 1.1 (0.43-8.2) | 4.2 (0.23-5.9) | 35 (6.1-35) |
| High grade | 2.2 (0.93-21) | 4.1 (0.69-5.7) | 2.1 (1.3-4) |
| p-value | 0.0111 | 0.3832 | <0.0001 |
| stages | | | |
| muscle-invasive (T2-T4) | 1.7 (0.88-19) | 2.6 (-0.36-5.1) | 2.2 (1.9-4.7) |
| non -muscle- invasive (Ta-T1) | 2.2 (0.57-8.2) | 4.2 (1.4-6) | 2.5 (1.1-6.1) |
| p-value | 0.9289 | 0.0818 | 0.0195 |

Example 5 Detection of the co-methylation of 22 methylated regions in urine DNA samples.

The co-methylation level of 22 methylated regions were detected for the urine DNA samples from the bladder cancer group, the benign urological disease group, and the healthy group to identify the use of these methylated regions of urine samples in identifying the incidence and typing of bladder cancer. Among them, there are 70 urine samples from patients with bladder cancer, 49 urine samples from patients with benign urinary diseases (including urinary tract stones, urinary tract infection, prostatic hyperplasia, glandular cystitis, etc.) and 5 urine samples from healthy people (urine routine examination/ultrasonic examination of the urinary system showed normal results and no other tumors were suspected). The pathological and clinical information composition of all samples is shown in table 9.

**Table 9 Pathological and clinical composition information of urine DNA samples**

| | Bladder cancer group | Benign urinary disease group | healthy group |
|---|---|---|---|
| cases | 70 | 49 | 5 |
| Average age (range) | 63 (26-83) | 58 (10-90) | |
| gender: cases (proportion) | | | |
| male | 56 (80%) | 33 (67.3%) | 4 (80%) |
| female | 14 (20%) | 16 (32.7%) | 1 (20%) |
| grade : cases (proportion) | | | |
| preinvasive carcinoma | 2 (2.9%) | | |
| Low grade | 12 (17.1%) | | |
| High grade | 56 (80%) | | |
| stage : cases (proportion) | | | |
| Ta | 14 (20%) | | |
| T1 | 19 (27.1%) | | |
| T2-T4 | 31 (44.3%) | | |
| Invasiveness: cases (proportion) | | | |
| Muscle-invasive (T2-T4) | 28 (40%) | | |
| Non- muscle-invasive (Ta-T1) | 40 (57.1%) | | |

The co-methylation level of 22 methylated regions was detected for DNA from the 124 urine samples according to the detection method as described in example 3. The C_{T} values for each methylated region from the detection were corrected by the C_{T} value for internal reference, and the relative cycle number of the target regions was obtained as d-C_{T} = C_{T} (target region) - C_{T} (internal reference). If the target regions are undetectable, the relative cycle number of the target regions is given as d- C_{T} = 35..

The median relative cycle number d- C_{T} values and their interquartile range for the co-methylation of the 22 methylated regions of the DNA from 124 urine samples were shown in table 10. The p-value according to comparison of difference between single methylated regions in different groups is also shown in table 10, in which the standard for statistically significant difference between two groups is p<0.05.The methylation heat map of 22 methylated regions of 124 samples according to the d- C_{T} values is shown in Figure 2.

**Table 10 analysis of the relative cycle number d-C_{T} values and interquartile range of d-C_{T} values for the co-methylation level in the 22 methylated regions of urine DNA samples from bladder cancer group, benign urological disease group and healthy group, and difference in different groups.**

| | median d-C_{T} (interquartile range of d-C_{T} value) | | | p-value (Bladder cancer - benign urological diseases) | p-value (Bladder cancer - healthy group) | p-value (Bladder cancer - noncancerous group) |
|---|---|---|---|---|---|---|
| | bladder cancer | benign urological diseases | healthy group | | | |
| SEQID NO. 1 | 2.1(-0.22-5.7) | 12.5(8.2-35) | 35(22.1-35) | <0.0001 | <0.0001 | <0.0001 |
| SEQID NO. 2 | 13.1(6.7-35) | 35(35) | 35(35) | <0.0001 | 0.0025 | <0.0001 |
| SEQID NO. 3 | 3.8(-0.66-35) | 35(35) | 35(35) | <0.0001 | <0.0001 | <0.0001 |
| SEQID NO. 4 | 4.5(2.9-8.5) | 35(35) | 35(35) | <0.0001 | <0.0001 | <0.0001 |
| SEQID | 3.1(1-7) | 35(9-35) | 35(22.8-35) | <0.0001 | <0.0001 | <0.0001 |
| NO. 5 | | | | | | |
| SEQID NO. 6 | 3.5(0.63-7.8) | 12.4(8.6-35) | 35(23-35) | <0.0001 | <0.0001 | <0.0001 |
| SEQID NO. 7 | 4.4(0.84-4.4) | 7.9(5.7-18.4) | 12(8.4-35) | <0.0001 | 0.0010 | <0.0001 |
| SEQID NO. 8 | 4.3(0.5-9.1) | 10.4(7.6-19.3) | 35(35) | <0.0001 | 0.0001 | <0.0001 |
| SEQID NO. 9 | 3.1(0.16-7) | 10(6.9-35) | 35(10.3-35) | <0.0001 | 0.0001 | <0.0001 |
| SEQID NO.10 | 3(-0.45-7.1) | 9.2(7.7-11.9) | 8.4(7.7-10.8) | 0.0001 | 0.3258 | 0.0001 |
| SEQIDN O.11 | 2.1(-0.66-6.3) | 7.8(6.1-9.8) | 12.8(10.2-24.4) | 0.0002 | 0.0059 | <0.0001 |
| SEQID NO.12 | 35(7.5-35) | 35(35) | 35(35) | 0.0007 | 0.1097 | 0.0004 |
| SEQID NO.13 | 6(2.7-35) | 7.5(5.4-35) | 4.8(3.1-6.3) | 0.3540 | 0.0275 | 0.1655 |
| SEQID NO.14 | 7.7(4.2-35) | 35(35) | 8.6(7.9-22.2) | 0.0001 | 0.8913 | 0.0004 |
| SEQID NO.15 | 13.8(7.9-20.1) | 20.8(14.4-35) | 19.5(15.9-21.1) | 0.0013 | 0.6322 | 0.0018 |
| SEQID NO.16 | 1.5(-1.4-4.2) | 5.1(3.8-6) | 6.6(6-7.2) | 0.0591 | 0.2963 | 0.0438 |
| SEQID NO.17 | 5.4(2.6-12.6) | 35(12.2-35) | 35(23.5-35) | <0.0001 | 0.0002 | <0.0001 |
| SEQID NO.18 | 4.4(1.4-6.2) | 7.6(5.2-9) | 11.2(8-23.6) | 0.0588 | 0.0465 | 0.0251 |
| SEQID NO.19 | 5(2.1-10.4) | 35(9.5-35) | 35(35) | <0.0001 | <0.0001 | <0.0001 |
| SEQID NO.20 | 6.7(2.1-35) | 35(13.7-35) | 35(35) | <0.0001 | <0.0001 | <0.0001 |
| SEQID NO.21 | 6.2(3.7-7.7) | 8.6(7.6-9.8) | 9(8.1-9.4) | 0.1601 | 0.9104 | 0.1788 |
| SEQID NO.22 | 0.65(-1.5-3.5) | 3.6(1.8-5.2) | -0.54(-0.87-0.72) | 0.1339 | 0.8549 | 0.1727 |

As shown in figure 2 and table 10, the median relative cycle number d-C_{T} values for the co-methylation level of the 22 methylated regions of urine DNA samples from bladder cancer group is significantly lower than that of the benign urological disease group (p < 0.05), and thus it can be concluded that the co-methylation level of the selected 22 methylated regions is significantly higher in urine DNA of bladder cancer group, positively correlated with the development of bladder cancer, and can be used as a biomarker for identifying the occurrence of bladder cancer based on a urine sample. Meanwhile, the bladder cancer group and healthy group are significantly differentiated in a plurality of methylated regions. Thus, these methylated regions also can be used as a biomarker for identifying the occurrence of bladder cancer based on a urine sample. In combination with the results from Example 4, the application of the 22 methylated regions in identifying the occurrence of bladder cancer can be used for both tissue samples and urine samples, with similar results.

In addition, according to analysis on the d-C_{T} value for 22 methylated regions at different levels and stages according to patient pathological information of bladder cancer urine samples, it was found that different grade or different stages can be significantly differentiated in some methylated regions (p < 0.05), which demonstrates that these methylated regions and their combinations could be further used as biomarkers for identifying the bladder cancer at different grades or different stages based on urine DNA. The methylated regions are listed in table 11, and the values as shown are the median d-C_{T} values for these methylated regions of respective groups (and interquartile range of these d-C_{T} values).

**Table 11 Median d-C_{T} values (interquartile range, IQR) for the different methylated regions of bladder cancer at different grades and stages in urine sample group**

| | SEQ ID NO. 2 | SEQ ID NO. 3 | SEQ ID NO. 6 | SEQ ID NO. 13 | SEQ ID NO. 14 | SEQ ID NO. 15 | SEQ ID NO. 17 |
|---|---|---|---|---|---|---|---|
| Grade | | | | | | | |
| Low grade | 35(9.6-35) | 35 (3.3-35) | 6.2(2.4-12.1) | 5.7(2.7-35) | 13.8 (7.7-35) | 20.5(7.6-35) | 35(11.4-35) |
| High grade | 11.4(6.1-35) | 3.5(-1.2-10..7) | 3.5(0.2-7.5) | 6.1(2.4-35) | 6.6 (4-28.6) | 12.9(8-17.5) | 4.1(2.4-8.6) |
| p-value | 0.033 | 0.0016 | 0.3136 | 0.0666 | 0.0215 | 0.0207 | <0.0001 |
| Stages | | | | | | | |
| Muscle-invasive (T2-T4) | 9.3 (6-35) | 3.1(-1.7-9.1) | 3(0.35-5.8) | 35(2.6-35) | 6.6 (3.2-35) | 13.1(9.5-17.2) | 4.7(2.3-8.8) |
| Non-muscle-invasive (Ta-Tl) | 35 (35) | 5.2(-0.66-35) | 5(1.1-10.2) | 5(2.5-35) | 8.4 (4.2-35) | 14.2(0.78-29.5) | 5.9(2.8-35) |
| p-value | 0.0011 | 0.0126 | 0.0493 | 0.0032 | 0.2938 | 0.458 | 0.0912 |

Meanwhile, according to the combination scheme of example 3, the detection method described in this example can be used for the parallel detection of 2-22 methylated regions, and the detection method is flexible, simple and feasible for the combination and collocation of methylated regions.

### Example 6

parallel co-methylation detection of 1-3 methylated regions in 22 methylated regions

When the parallel co-methylation detection of the target methylated regions is carried out on 1-3 methylated regions of the 22 methylated regions, by using the combination scheme in example 3, the detection method can be adopted. The specific detection process is as follows:
1. DNA exctration;
DNA extraction kit was purchased from QIAGEN company and DNA extraction was carried out according to the instruction of the extraction kit.
2. DNA conversion with bisulfite
DNA bisulfite conversion kit was purchased from Zymo and DNA bisulfite conversion was carried out according to the instructions of the kit.
3. fluorescence Quantitative PCR assay
Primers and probes for 1-3 methylated regions and internal reference were selected and assay was carried out in one reaction well (primer and probe sequences are shown in table 2, and the combination scheme of methylated regions is shown in example 3).
1) Preparation of qPCR reaction solution: PCR mixture was prepared according to table 12, without adding DNA therein.

**Table 12 Preparation of qPCR reaction solution**

| Reagent | volume (µL) |
|---|---|
| DEPC water | 2.8 |
| 2 X PCR Master Mix | 10.0 |
| primer and probe mixture for one or two or three markers as described in example 3 | 0.60 (each type) |
| 50X ROX | 0.40 |
| volume [µl] | 15.00 |

2) Adding DNA samples: into the PCR reaction well, added was 15 µL PCR mixture, followed by 2µL converted DNA which has a loading amount of 25 ng before conversion, wherein the concerted product was put into a PCR reaction well; the PCR reaction system had a total volume of 20 µL. Eddy oscillation and centrifugation.
3) procedure for PCR reaction: 95°C for 5 minutes; 60 cycles of 95°C for 15 seconds, 62°C for 40 seconds (fluorescent signal was collected at 62°C).
4. Data Processing and Analysis
The C_{T} values for each methylated region from the detection of the target regions were corrected by the C_{T} value for internal reference, and the relative cycle number of the target regions was obtained as d-C_{T} = C_{T} (target region) - C_{T} (internal reference). If the target regions are undetectable, the relative cycle number of the target regions is given as d- C_{T} = 35.

Taking the detection of positive control with combinations A and L of primers and probes for the methylated regions (example 3) as an example, according to the detection method described in example 3, comparison between relative cycle number d-C_{T} value with that in Example 3 is shown in table 13.

**Table 13 Comparison of d-C_{T} values from parallel co-methylation detection of 1-3 methylated regions in positive control with that from detection method of 22 methylated regions**

| | d-C_{T} values from parallel detection of 1-3 methylated regions | d-C_{T} values from detection method of 22 methylated regions |
|---|---|---|
| SEQ ID NO.1 | 2.48 | 2.19 |
| SEQ ID NO.2 | 3.40 | 4.17 |
| SEQ ID NO.3 | -0.58 | -1.03 |
| SEQ ID NO.4 | -3.09 | -3.89 |

The results in table 13 show that the d-C_{T} values detected by the method in this example is highly consistent with the d-C_{T} values detected by the detection method for 22 methylated regions (example 3). The analysis on correlation between the d-C_{T} values in these regions resulted from the two detection methods showed that the coefficient of correlation is R = 0.995 (Pearson R), and thus it can be determined that there was no difference in the detection of the co-methylation level of the same methylated region between the two detection methods. When the target methylated regions in the parallel co-methylation detection is 1-3 methylated regions of the 22 methylated regions, the detection method described in this example may allow reduced steps for pre-amplifying target fragments by multiplex PCR, making the parallel detection of less than 4 methylated regions more convenient and quick.

### Example 7

The mathematical modeling analysis of methylated region combination was carried out for the relative cycle number d-C_{T} value for co-methylation of 22 methylated regions (SEQ ID no. 1-22) in 124 urine DNA samples obtained in Example 5, to explore the application of 22 methylated regions as a combination of biomarkers in the detection of the occurrence of staging of bladder cancer and to identify the superiority of performance by comparing with a single methylated region as a marker.

First of all, the pathological and clinical information of 124 urine samples was compared. According to the relative cycle number d-C_{T} value for co-methylation of 22 methylated regions in bladder cancer group and non -bladder cancer group (including urinary benign disease group and healthy group) in contrast with pathology, the ROC curve of diagnostic model for identifying the occurrence of bladder cancer based on a single methylated region was established. The AUC value was calculated and identifying threshold value was drawn for this region according to the ROC curve. The sensitivity, specificity and Youden index for this methylated region were calculated according to the threshold value in contrast with pathology. At the same time, according to the relative cycle number d-C_{T} value for co-methylation in 22 methylated regions, 2-22 methylation markers were selected for logistic regression fitting. The fitted equation can be used to calculate the risk score of bladder cancer of each sample to identify the occurrence of bladder cancer. According to the different combinations of 2-22 methylated regions, multiple logistic regression models and equations can be generated for identifying the occurrence of bladder cancer. The sensitivity, specificity, AUC and Youden index for the methylated region combinations were obtained based on the risk scores of bladder cancer calculated from these equations in contrast with pathology. Table 14 shows the comparison of performance parameters for identifying the occurrence of bladder cancer between the combined models and the single methylated region. In addition, figure 3 shows the distribution of risk scores in bladder cancer and non-bladder cancer groups using a combined model of 8 methylated regions of the 22 methylated regions (SEQ ID no.1-8).

As shown in Figure 3, the risk scores of bladder cancer obtained in a combined identifying model of 8 methylated regions can discriminate obviously the bladder cancer group from the non-bladder cancer group, further demonstrating that the combination of these methylated regions can be used as a marker combination for identifying the occurrence of bladder cancer.

From comparison of diagnostic efficacy in table 14, it can be seen that using a single methylated region as the diagnostic model exhibits a lower diagnostic performance than the combined model of multiple methylated regions. The combination of 2-22 methylated regions has a higher sensitivity in identifying the occurrence of bladder cancer, and has a significantly higher Youden index, i.e. an overall performance parameter reflecting the sensitivity and specificity, than identification by a single methylated region, with superior identifying advantage.

**Table 14 comparison of a model of single methylated region with a model of multiple methylated regions for the diagnosis of bladder cancer**

| Combination of the regions (SEQ ID NO.) | AUC | sensitivity | specificity | Youden index |
|---|---|---|---|---|
| 1 | 0.84 | 70% | 96% | 0.66 |
| 1+2 | 0.89 | 80% | 91% | 0.71 |
| 1+2+3 | 0.89 | 89% | 85% | 0.75 |
| 1-8 | 0.89 | 84% | 85% | 0.69 |
| 1-22 | 0.88 | 81% | 89% | 0.70 |

Furthermore, the sensitivity of the combined model of 2-22 methylated regions for bladder cancer at different grades and stages is compared with that of a single methylated region, as shown in table 15.

**Table 15 sensitivity comparison for bladder cancer at different stages and grades between the model of single methylated region and the model of multiple methylated regions**

| Combination of the regions (SEQ ID NO) | High -grade bladder cancer | Low-grade bladder cancer | Invasive bladder cancer | Non-invasive bladder cancer |
|---|---|---|---|---|
| 1 | 86% | 54% | 87% | 80% |
| 1+2 | 88% | 70% | 91% | 84% |
| 1+2+3 | 87% | 75% | 88% | 82% |
| 1-8 | 89% | 70% | 88% | 85% |
| 1-22 | 89% | 75% | 89% | 83% |

The data in table 15 further shows that the sensitivity of the combined model of 2-22 methylated regions has a higher sensitivity for identifying high-grade, low-grade, invasive and non-invasive bladder cancer than the model of single methylation, indicating that the combined model of 2-22 methylated regions can be used to identify these groups of bladder cancer.

In addition, 2-22 methylated regions were selected to carry out mathematical modelling for high-grade and low-grade bladder cancer groups or invasive and non -invasive bladder cancer groups. Random forest algorithm was used to select the methylated region combination that are capable of making identification, then the identifying threshold value for each methylated region was set to obtain identifying models. According to these identifying models, the bladder cancer group could be classified (high-grade or low-grade) or staged (invasive or non-invasive). The sensitivity, specificity and Youden index can be obtained in contrast with the pathological results. Sensitivity comparison for different grades or stages with the model of single methylated region is shown in table 16.

**Table 16 Sensitivity comparison for bladder cancer at different grades or stages between the model of multiple methylated region and the model of single methylated region**

| Combination of the regions (SEQ ID NO.) | Sensitivity | specificity | Youden index |
|---|---|---|---|
| Distinguishing invasive bladder cancer from non-invasive bladder cancer | | | |
| 22 | 88.5% | 39% | 0.275 |
| 13 | 96.2% | 19.5% | 0.157 |
| 22+13+10 | 88.5% | 68.3% | 0.568 |
| 15+13+6+16+18 | 88.5% | 78% | 0.665 |
| 15+13+6+16+18+10 | 88.5% | 80.5% | 0.690 |

| Distinguishing high-grade bladder cancer from low-grade bladder cancer | | | |
|---|---|---|---|
| 17 | 85.7% | 72.7% | 0.584 |
| 22 | 85.7% | 45.5% | 0.312 |
| 17+5+13 | 87.5% | 90.9% | 0.784 |
| 17+22+13 | 87.5% | 90.9% | 0.784 |

According to comparison in the grading and staging diagnostic efficiency in table 16, using a single methylated region as a grading and staging diagnostic model exhibits a lower diagnostic performance than the combined model of multiple methylated regions, and the combination of 2-22 methylated regions has a higher sensitivity and specificity in identifying the different grade and stage of bladder cancer, and has a significantly higher Youden index, i.e. an overall performance parameter reflecting the sensitivity and specificity, than identification by a single methylated region, with superior identifying advantage. In addition, it also shows that the combination of multiple methylated regions in these 22 methylated regions can be used for further grading and staging of bladder cancer, which has a more accurate guiding significance for diagnosis scheme and drug guidance for bladder cancer. The technical features of the above-mentioned embodiments can be arbitrarily combined. For simple description, not all of the possible combinations of the technical features in the embodiments above are described. However, as long as the combination of the technical features have no conflict or contradiction collision with each other, it should be considered as part of the scope of the specification herein.

Only a few embodiments are herein elaborated in a specific and detailed manner, and cannot be construed as limiting the scope of the present application. It should be noted that for ordinary technicians in the field, certain modifications and improvements can be made without departing from the spirit of the present disclosure, which are within the protection scope of the invention. Therefore, the scope of protection of the invention patent shall be

## Claims

1. A combination of DNA methylation markers for bladder cancer detection, wherein the combination of DNA methylation markers is selected from any combination of two or more sequences from SEQ ID No. 1 to SEQ ID No. 22 of the co-methylated regions indicated by [CG], or is selected from any combination of two or more completely complementary sequences to SEQ ID No. 1 to SEQ ID No. 22.

2. The combination of DNA methylation biomarkers for bladder cancer detection according to claim 1, wherein the combination of DNA methylation markers for bladder cancer detection includes a combination of at least two sequences selected from SEQ ID No. 2, SEQ ID No. 3, SEQ ID No. 6, SEQ ID No. 13, SEQ ID No. 14, SEQ ID No. 15, SEQ ID No. 17, or a combination of at least two completely complementary sequences to SEQ ID No. 2, SEQ ID No. 3, SEQ ID No. 6, SEQ ID NO. 13, SEQ ID NO. 14, SEQ ID NO. 15, SEQ ID NO. 17.

3. The combination of DNA methylation markers for bladder cancer detection according to claim 1, wherein the combination of DNA methylation markers for bladder cancer detection is a combination of SEQ ID No. 1 and SEQ ID No.2 or a combination of completely complementary sequences to SEQ ID No. 1 and SEQ ID No. 2 .

4. The combination of DNA methylation markers for bladder cancer detection according to claim 3, wherein the combination of DNA methylation markers for bladder cancer detection is a combination of SEQ ID No. 1, SEQ ID No. 2 and SEQ ID No.3, or a combination of completely complementary sequences to SEQ ID No. 1, SEQ ID No. 2 and SEQ ID No.3.

5. The combination of DNA methylation markers for bladder cancer detection according to claim 4, wherein the combination of DNA methylation markers for bladder cancer detection is a combination of SEQ ID Nos. 1-8 or a combination of completely complementary sequences to SEQ ID Nos. 1-8.

6. The combination of DNA methylation markers for bladder cancer detection according to claim 1, wherein the combination of DNA methylation markers for bladder cancer detection is a combination of SEQ ID Nos. 1-22 or a combination of completely complementary sequences to SEQ ID Nos. 1-22.

7. The combination of DNA methylation markers for bladder cancer detection according to claim 1, wherein the combination of DNA methylation markers for bladder cancer detection comprises the following groups :
a combination of SEQ ID No.2 and SEQ ID No. 1,
a combination of SEQ ID NO. 17, SEQ ID NO. 20 and SEQ ID NO.1,
a combination of SEQ ID NO.2, SEQ ID NO. 9 and SEQ ID NO.8,
a combination of SEQ ID NO.15 and SEQ ID NO.6,
a combination of SEQ ID NO.3, SEQ ID NO. 18 and SEQ ID NO. 14,
a combination of SEQ ID NO.16 and SEQ ID NO.12,
a combination of SEQ ID NO.22, SEQ ID NO. 5 and SEQ ID NO. 4,
a combination of SEQ ID NO.13, SEQ ID NO. 10 and SEQ ID NO. 21,
a combination of SEQ ID NO. 19, SEQ ID NO. 11 and SEQ ID NO. 7,
a combination of SEQ ID NO.2 and SEQ ID NO.6,
a combination of SEQ ID NO.3 and SEQ ID NO.4, or a combination of completely complementary sequences to the above combination of the DNA methylation markers.

8. Use of the combination of DNA methylation markers for bladder cancer detection according to any one of claims 1-7 in the preparation of a kit for detection, diagnosis, classification or prediction, treatment monitoring, prognosis or otherwise evaluation of bladder cancer.

9. A kit for identifying bladder cancer at different grades or stages, wherein the kit comprises a reagent for detecting a co-methylation level of a combination of DNA methylation markers of at least two of SEQ ID No.2, SEQ ID No.3, SEQ ID No.5, SEQ ID No.6, SEQ ID No.13, SEQ ID No.14, SEQ ID No.15, SEQ ID No.16, SEQ ID No.17, SEQ ID No.18, SEQ ID No.21, and SEQ ID No.22 of a co-methylated region indicated by [CG], or a co-methylation level of a combination of completely complementary sequences to SEQ ID No.2, SEQ ID No.3, SEQ ID No.5, SEQ ID No.6, SEQ ID No. 13, SEQ ID No.14, SEQ ID No.15, SEQ ID No.16, SEQ ID No.17, SEQ ID No.18, SEQ ID No.21, and SEQ ID No.22.

10. The kit for identifying bladder cancer at different grades or stages according to claim 9, wherein the combination of DNA methylation markers for bladder cancer detection is a combination of DNA methylation markers of SEQ ID No.6, SEQ ID No.13, SEQ ID No.16, and SEQ ID No.18 or a combination of DNA methylation markers of completely complementary sequences to SEQ ID No.6, SEQ ID No. 13, SEQ ID No.16, and SEQ ID No.18.

11. The kit for identifying bladder cancer at different grades or stages according to claim 10, wherein the combination of DNA methylation markers for bladder cancer detection comprises DNA methylation markers of SEQ ID NO.6, SEQ ID NO.10, SEQ ID NO.13, SEQ ID NO. 16, and SEQ ID NO. 18 or DNA methylation markers of complementary sequences to SEQ ID NO.6, SEQ ID NO.10, SEQ ID NO.13, SEQ ID NO. 16, and SEQ ID NO. 18.

12. The kit for identifying bladder cancer at different grades or stages according to claim 9, wherein the combinations of DNA methylation markers for bladder cancer detection comprises DNA methylation markers of SEQ ID NO.17 and SEQ ID NO.13 or DNA methylation markers of completely complementary sequences to SEQ ID NO.17 and SEQ ID NO.13.

13. The kit for identifying bladder cancer at different grades or stages according to claim 12, wherein the combination of DNA methylation markers is a combination of SEQ ID NO. 17, SEQ ID NO.13, and SEQ ID NO.5, or a combination of complementary sequences to SEQ ID NO.17, SEQ ID NO.13, and SEQ ID NO.5; or the combination of DNA methylation markers is a combination of SEQ ID NO.17, SEQ ID NO.13 and SEQ ID NO.22 or a combination of the complementary sequences to SEQ ID NO. 17, SEQ ID NO.13 and SEQ ID NO.22.

14. The kit for bladder cancer detection, comprising the reagent for detecting the methylation levels of the combination of DNA methylation markers according to any one of claims 1-7.

15. The kit for bladder cancer detection according to claim 14, wherein when fluorescence quantitative PCR is used, the kit for bladder cancer detection comprises amplification primers and fluorescent probes for each methylated region, the amplification primers and fluorescent probes being selected from:
SEQ ID NO. 23, SEQ ID NO. 45 and SEQ ID NO. 67 for SEQ ID NO.1;
SEQ ID NO. 24, SEQ ID NO. 46 and SEQ ID NO. 68 for SEQ ID NO.2;
SEQ ID NO. 25, SEQ ID NO. 47 and SEQ ID NO. 69 for SEQ ID NO.3;
SEQ ID NO. 26, SEQ ID NO. 48 and SEQ ID NO. 70 for SEQ ID NO.4;
SEQ ID NO. 27, SEQ ID NO. 49 and SEQ ID NO. 71 for SEQ ID NO.5;
SEQ ID NO. 28, SEQ ID NO. 50 and SEQ ID NO. 72 for SEQ ID NO.6;
SEQ ID NO. 29, SEQ ID NO. 51 and SEQ ID NO. 73 for SEQ ID NO.7;
SEQ ID NO. 30, SEQ ID NO. 52 and SEQ ID NO. 74 for SEQ ID NO.8;
SEQ ID NO. 31, SEQ ID NO. 53 and SEQ ID NO. 75 for SEQ ID NO.9;
SEQ ID NO. 32, SEQ ID NO. 54 and SEQ ID NO. 76 for SEQ ID NO.10;
SEQ ID NO. 33, SEQ ID NO. 55 and SEQ ID NO. 77 for SEQ ID NO.11;
SEQ ID NO. 34, SEQ ID NO. 56 and SEQ ID NO. 78 for SEQ ID NO.12;
SEQ ID NO. 35, SEQ ID NO. 57 and SEQ ID NO. 79 for SEQ ID NO.13;
SEQ ID NO. 36, SEQ ID NO. 58 and SEQ ID NO. 80 for SEQ ID NO.14;
SEQ ID NO. 37, SEQ ID NO. 59 and SEQ ID NO. 81 for SEQ ID NO.15;
SEQ ID NO. 38, SEQ ID NO. 60 and SEQ ID NO. 82 for SEQ ID NO.16;
SEQ ID NO. 39, SEQ ID NO. 61 and SEQ ID NO. 83 for SEQ ID NO.17;
SEQ ID NO. 40, SEQ ID NO. 62 and SEQ ID NO. 84 for SEQ ID NO.18;
SEQ ID NO. 41, SEQ ID NO. 63 and SEQ ID NO. 85 for SEQ ID NO.19;
SEQ ID NO. 42, SEQ ID NO. 64 and SEQ ID NO. 86 for SEQ ID NO.20;
SEQ ID NO. 43, SEQ ID NO. 65 and SEQ ID NO. 87 for SEQ ID NO.21; or
SEQ ID NO. 44, SEQ ID NO. 66 and SEQ ID NO. 88 for SEQ ID NO.22; or
primers and probes having a plurality of consecutive nucleotides which are at least 70%, 80%, 90%, 95%, or 99% identical to the above sequences.

16. The kit for bladder cancer detection according to claim 14, wherein when fluorescent quantitative PCR is used, the kit for bladder cancer detection comprises amplification primers and fluorescent probes for each methylated region, the amplification primers and fluorescent probes being selected from:
SEQ ID NO. 89, SEQ ID NO. 111 and SEQ ID NO. 133 for SEQ ID NO.1;
SEQ ID NO. 90, SEQ ID NO. 112 and SEQ ID NO. 134 for SEQ ID NO.2;
SEQ ID NO. 91, SEQ ID NO. 113 and SEQ ID NO. 135 for SEQ ID NO.3;
SEQ ID NO. 92, SEQ ID NO. 114 and SEQ ID NO. 136 for SEQ ID NO.4;
SEQ ID NO. 93, SEQ ID NO. 115 and SEQ ID NO. 137 for SEQ ID NO.5;
SEQ ID NO. 94, SEQ ID NO. 116 and SEQ ID NO. 138 for SEQ ID NO.6;
SEQ ID NO. 95, SEQ ID NO. 117 and SEQ ID NO. 139 for SEQ ID NO.7;
SEQ ID NO. 96, SEQ ID NO. 118 and SEQ ID NO. 140 for SEQ ID NO.8;
SEQ ID NO. 97, SEQ ID NO. 119 and SEQ ID NO. 141 for SEQ ID NO.9;
SEQ ID NO. 98, SEQ ID NO. 120 and SEQ ID NO. 142 for SEQ ID NO.10;
SEQ ID NO. 99, SEQ ID NO. 121 and SEQ ID NO. 143 for SEQ ID NO.11;
SEQ ID NO. 100, SEQ ID NO. 122 and SEQ ID NO. 144 for SEQ ID NO.12;
SEQ ID NO. 101, SEQ ID NO. 123 and SEQ ID NO. 145 for SEQ ID NO.13;
SEQ ID NO. 101, SEQ ID NO. 124 and SEQ ID NO. 146 for SEQ ID NO.14;
SEQ ID NO. 103, SEQ ID NO. 125 and SEQ ID NO. 147 for SEQ ID NO.15;
SEQ ID NO. 104, SEQ ID NO. 126 and SEQ ID NO. 148 for SEQ ID NO.16;
SEQ ID NO. 105, SEQ ID NO. 127 and SEQ ID NO. 149 for SEQ ID NO.17;
SEQ ID NO. 106, SEQ ID NO. 128 and SEQ ID NO. 150 for SEQ ID NO.18;
SEQ ID NO. 107, SEQ ID NO. 129 and SEQ ID NO. 151 for SEQ ID NO.19;
SEQ ID NO. 108, SEQ ID NO. 130 and SEQ ID NO. 152 for SEQ ID NO.20;
SEQ ID NO. 109, SEQ ID NO. 131 and SEQ ID NO. 153 for SEQ ID NO.21; or
SEQ ID NO. 110, SEQ ID NO. 132 and SEQ ID NO. 154 for SEQ ID NO.22; or
primers and probes having a plurality of consecutive nucleotides which are at least 70%, 80%, 90%, 95%, or 99% identical to the above sequences.

17. The kit for bladder cancer detection according to claim 14, wherein when fluorescence quantitative PCR is used, the kit for bladder cancer detection comprises amplification primers and fluorescent probes for each methylated region, the amplification primers and fluorescent probes being selected from:
SEQ ID NO. 155, SEQ ID NO. 177 and SEQ ID NO. 199 for SEQ ID NO. 1 ;
SEQ ID NO. 156, SEQ ID NO. 178 and SEQ ID NO. 200 for SEQ ID NO.2 ;
SEQ ID NO. 157, SEQ ID NO. 179 and SEQ ID NO. 201for SEQ ID NO.3 ;
SEQ ID NO. 158, SEQ ID NO. 180 and SEQ ID NO. 202 for SEQ ID NO.4 ;
SEQ ID NO. 159, SEQ ID NO. 181 and SEQ ID NO. 203 for SEQ ID NO.5;
SEQ ID NO. 160, SEQ ID NO. 182 and SEQ ID NO. 204 for SEQ ID NO.6 ;
SEQ ID NO. 161, SEQ ID NO. 183 and SEQ ID NO. 205 for SEQ ID NO.7 ;
SEQ ID NO. 162, SEQ ID NO. 184 and SEQ ID NO. 206 for SEQ ID NO.8 ;
SEQ ID NO. 163, SEQ ID NO. 185 and SEQ ID NO. 207 for SEQ ID NO.9 ;
SEQ ID NO. 164, SEQ ID NO. 186 and SEQ ID NO. 208 for SEQ ID NO.10 ;
SEQ ID NO. 165, SEQ ID NO. 187 and SEQ ID NO. 209 for SEQ ID NO.11 ;
SEQ ID NO. 166, SEQ ID NO. 188 and SEQ ID NO. 210 for SEQ ID NO.12 ;
SEQ ID NO. 167, SEQ ID NO. 189 and SEQ ID NO. 211 for SEQ ID NO.13 ;
SEQ ID NO. 168, SEQ ID NO. 190 and SEQ ID NO. 212 for SEQ ID NO.14 ;
SEQ ID NO. 169, SEQ ID NO. 191 and SEQ ID NO. 213 for SEQ ID NO.15 ;
SEQ ID NO. 170, SEQ ID NO. 192 and SEQ ID NO. 214 for SEQ ID NO.16 ;
SEQ ID NO. 171, SEQ ID NO. 193 and SEQ ID NO. 215 for SEQ ID NO.17 ;
SEQ ID NO. 172, SEQ ID NO. 194 and SEQ ID NO. 216 for SEQ ID NO.18 ;
SEQ ID NO. 173, SEQ ID NO. 195 and SEQ ID NO. 217 for SEQ ID NO.19 ;
SEQ ID NO. 174, SEQ ID NO. 196 and SEQ ID NO. 218 for SEQ ID NO.20 ;
SEQ ID NO. 175, SEQ ID NO. 197 and SEQ ID NO. 219 for SEQ ID NO.21 ; or
SEQ ID NO. 176, SEQ ID NO. 198 and SEQ ID NO. 220 for SEQ ID NO.22 ; or
primers and probes having a plurality of consecutive nucleotides which are at least 70%, 80%, 90%, 95%, or 99% identical to the above sequences.

18. The kit for bladder cancer detection according to any one of claims 14-17, further comprising primers and probes for internal reference gene: SEQ ID Nos.221-223; or primers or probes having a plurality of consecutive nucleotides which are at least 70%, 80%, 90%, 95%, or 99% identical to the above sequences.

19. A method for bladder cancer detection, wherein the method comprises:
extracting genomic DNA and / or free DNA from a biological sample to be detected;
performing bisulfite conversion of the DNA;
detecting co-methylation of the combination of DNA methylation markers according to claim 1 of the bisulfite-converted DNA and a control, to obtain a methylation profile,
comparing the methylation profile of the combination of DNA methylation markers with the identifying threshold of a profile obtained from mathematical modelling based on datasets to identify the presence of bladder cancer in the biological sample.

20. The method for bladder cancer detection according to claim 19, wherein methods for co-methylation detection comprises: methylation specific PCR, DNA methylation-based chip, targeted DNA methylation sequencing, digital PCR, quantitative and fluorescence quantitative PCR.

21. A method for diagnosis, staging and classification of bladder cancer, wherein the method comprises:
extracting genomic DNA and / or free DNA from biological sample to be detected;
performing bisulfite conversion of the DNA;
detecting co-methylation of the combination of DNA methylation markers according to claim 1 for the bisulfite-converted DNA;
comparing a relative number of cycles d-C_{T} of target DNA methylation marker regions andwith a pre-defined threshold,
identifying grade or stage of bladder cancer of the biological sample from different sources.

22. A method for prediction, treatment monitoring, prognosis or otherwise evaluation of bladder cancer, wherein the method comprises:
obtaining a biological sample from an individual;
extracting genomic DNA and / or free DNA from the biological sample;
performing bisulfite conversion of the DNA;
contacting the bisulfite-converted DNA with a plurality of reagents for specifically detecting co-methylation of DNA methylation markers according to the claim1 to measure a co-methylation level of the DNA methylation markers of the biological sample;
comparing with a identifying threshold for co-methylation level obtained from mathematical modelling based on datasets to identify prediction, treatment monitoring, or prognosis of bladder cancer.

23. The method according to any one of claims 19-22, wherein the biological sample is selected from blood, plasma, saliva or serum.

24. The method for detecting bladder cancer according to any one of claims 19-22, wherein the biological sample is tissue.

25. The method for detecting bladder cancer according to any one of claims 19-22, wherein the biological sample is selected from urine, exfoliated cells in urine, urine sediment or urine supernatant.
